(19) **Europäisches Patentamt / European Patent Office / Office européen des brevets**

(11) **EP 3 395 369 A1**

(12) **EUROPEAN PATENT APPLICATION**

(43) Date of publication:
**31.10.2018 Bulletin 2018/44**

(21) Application number: **18177254.2**

(22) Date of filing: **30.10.2015**

(51) Int Cl.:
*A61K 47/18* (2017.01)      *A61K 47/12* (2006.01)
*A61P 31/12* (2006.01)      *A61P 37/06* (2006.01)
*A61K 38/05* (2006.01)      *A61K 31/4164* (2006.01)
*A61K 31/519* (2006.01)      *A61K 31/522* (2006.01)
*A61K 31/7056* (2006.01)     *A61K 47/10* (2017.01)
*A61K 47/14* (2017.01)      *A61K 47/22* (2006.01)
*A61K 47/26* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.10.2014 JP 2014221315**

(62) Document number(s) of the earlier application(s) in accordance with Art. 76 EPC:
**15855750.4 / 3 213 769**

(71) Applicant: **ASAHI KASEI KABUSHIKI KAISHA Tokyo 101-8101 (JP)**

(72) Inventors:
• **Obae, Kazuhiro**
  **Tokyo, 101-8101 (JP)**

• **Yoshida, Naoya**
  **Tokyo, 101-8101 (JP)**
• **Kajihara, Kurumi**
  **Tokyo, 101-8101 (JP)**

(74) Representative: **Hoffmann Eitle**
**Patent- und Rechtsanwälte PartmbB**
**Arabellastraße 30**
**81925 München (DE)**

Remarks:
This application was filed on 12-06-2018 as a divisional application to the application mentioned under INID code 62.

(54) **TRANSDERMAL ABSORPTION ENHANCER AND TRANSDERMAL ABSORPTION ENHANCEMENT AID**

(57)    The present invention provides a transdermal preparation excellent in transdermal absorbability using various active ingredients by virtue of a transdermal absorption enhancement aid or a transdermal absorption enhancer comprising an equimolar salt of a) a basic functional group-containing compound having a molecular weight of from 50 to 120 and a melting point of from 50 to 350°C selected from guanidine or a derivative thereof, and b) an acidic functional group-containing compound having a LogP of -4 to 7.3.

EP 3 395 369 A1

## Description

Technical Field

The present invention relates to a transdermal absorption enhancer, in the field of pharmaceutical products, cosmetics, quasi-drugs, and the like, which is used for increasing absorbability (skin absorbability or transdermal absorbability) when an active ingredient (substance that exhibits physiological activity) is absorbed through the skin of humans or other animals (transdermally absorbed), and a transdermal absorption enhancement aid which is used, together with a transdermal absorption enhancer, for increasing the transdermal absorbability-improving effect of the transdermal absorption enhancer.

More specifically, the present invention relates to a transdermal absorption enhancer and a transdermal absorption enhancement aid which are effective for water-soluble active ingredients in addition to poorly soluble (less likely to be dissolved in water or an organic solvent) active ingredients.

Background Art

Methods for administering drugs to humans have generally been practiced through an oral route or injection. In recent years, research and development on transdermal administration have been active. This is because transdermal preparations have advantages such as avoidance of reduction in bioavailability caused by a first pass effect in the liver, and improvement in compliance for elderly people and children with dysphagia, as compared with oral preparations, and have advantages such as long term constancy of drug concentration in blood and reduction in invasiveness, as compared with injection preparations.

Heretofore, utilization of an ionic liquid has been proposed for the formulation of poorly soluble drugs, which are less likely to be dissolved in water or an organic solvent. As for the transdermal preparations, use of an ionic liquid has also been proposed for the dissolution of poorly soluble drugs.

Patent Literature 1 describes an ionic liquid that consists of an aliphatic quaternary ammonium cation consisting of a naturally occurring compound choline or a choline derivative and a carboxylic acid anion consisting of a biologically relevant compound and is in a liquid state at 90°C or lower. In Patent Literature 1, it is described that the ionic liquid is highly safe, and thus it can be applied to the medical field as a solubilizer for drugs, a fomentation, or the like, and can also be used *in vivo.* However, there is no disclosure indicating the solubility of drugs nor transdermal preparations.

Patent Literature 2 discloses 1-ethyl-3-methyl-1H-imidazolium trifluoromethanesulfonate salt as a solubilizer for poorly soluble drugs.

Patent Literature 3 describes (a) a Bronsted-type ionic liquid consisting of an acidic compound selected from levulinic acid, glyoxylic acid, glycolic acid, lactic acid, malic acid, citric acid, benzoic acid, salicylic acid, and glycine, and an organic amine compound selected from diethanolamine, triethanolamine, and triisopropanolamine, (b) an equimolar mixture of a quaternary ammonium salt compound selected from benzalkonium chloride and betaine, and a carboxylic acid compound selected from levulinic acid, glyoxylic acid, glycolic acid, lactic acid, malic acid, citric acid, benzoic acid, and salicylic acid, and (c) a Lewis-type ionic liquid selected from among 1-ethyl-3-methyl-1H-imidazolium trifluoromethanesulfonate salt and (BMI)Br. The literature also proposes use of an ionic liquid for enhancing the skin absorption of a drug in a transdermal preparation.

Patent Literature 4 describes (3) a transdermal absorption enhancement aid for an acidic drug or a basic drug, or a salt thereof, comprising, as an active ingredient, a fatty acid ionic liquid consisting of an equimolar salt of a fatty acid selected from levulinic acid, capric acid, isostearic acid, and oleic acid, and an organic amine selected from diethanolamine, triethanolamine, diisopropanolamine, and triisopropanolamine.

Non Patent Literature 1 describes a particular ionic liquid that dissolves a poorly soluble drug acyclovir (ACV) that serves as a therapeutic drug for viral infection, and states that the ionic liquid containing ACV dissolved therein formed microemulsions having an average particle size of 16.8 nm in isopropyl myristate in the presence of a surfactant and thereby transdermally penetrated.

Citation List

Non Patent Literature

Non Patent Literature 1: SCEJ 2nd Three-Branch Joint Meeting (Kitakyushu, 2009), Abstract

Patent Literature

Patent Literature 1: Japanese Patent Laid-Open No. 2008-162899
Patent Literature 2: Japanese Patent No. 4969065
Patent Literature 3: Japanese Patent Laid-Open No. 2012-233016
Patent Literature 4: Japanese Patent Laid-Open No. 2013-173805

Summary of Invention

Technical Problem

Many of the active ingredients in drugs (hereinafter, the active ingredient of a drug is also simply referred to as an "active ingredient") have significantly low transdermally absorption, as compared with an oral route or injection. This tendency is marked, particularly, for water-soluble active ingredients. This is because the stratum corneum of the skin is lipophilic and therefore exhibits a barrier function against the penetration of the water-soluble active ingredients.

For example, Patent Literatures 2 to 4 each disclose a transdermal preparation using an ionic liquid, but only poorly soluble active ingredients are listed as active ingredients and any water-soluble active ingredient is not described.

In the field of transdermal preparations, studies have been made on increase in the transdermal absorbability of an active ingredient into the stratum corneum.

For example, it is known that use of an organic solvent whose LogP, the logarithm of partition coefficient P between water and 1-octanol, is approximately 1 as a transdermal absorption enhancer is preferred for improvement in the transdermal absorbability of active ingredients. However, its effect is not sufficient.

There are many reports on use of transdermal absorption enhancers such as US3551554 (DMSO and N,N-DMF), Japanese Patent Laid-Open No. 51-32724, Japanese Patent Laid-Open No. 52-83914 (anionic or ampholytic surfactant), Japanese Patent Laid-Open No. 52-1035 (1-dodecylazacycloheptan-2-one and AZONE), Japanese Patent Laid-Open No. 2-193932 (terpene ketone such as l-carvone, menthone, and piperitone), Japanese Patent Laid-Open No. 2-207024 (d-limonene), glycols, fatty acids such as oleic acid, IPM, and fatty acid esters such as isopropyl palmitate, as to the enhancement of the transdermal absorption of water-soluble active ingredients.

However, these transdermal absorption enhancers have a limitation in dosage from the viewpoint of safety such as dermal irritation and handleability such as odor, and their transdermal absorption-enhancing effects are not always sufficient.

In addition, Non Patent Literature 1 discloses a transdermal drug delivery system achieved by dissolving a water-soluble drug acyclovir (ACV) in a particular ionic liquid and then making it into microemulsions in isopropyl myristate (IPM).

More specifically, it is described that the ACV dissolved in the ionic liquid could not be transdermally absorbed as it was, and could be transdermally absorbed only when the ionic liquid formed microemulsions (DLS particle size of ionic liquid/IPM: 16.8 nm) in IPM in the presence of a surfactant.

However, the transdermal absorbability of water-soluble active ingredients by use of the drug delivery system described in Non Patent Literature 1 is not sufficient. This is presumably because, while the intercellular space is reportedly 40 to 60 nm and particles need to have a size smaller than 1/5 of the space in order to pass through the space, the particle size of the microemulsion is larger than that.

Furthermore, the method described in Non Patent Literature 1 cannot form a transdermal preparation by merely dissolving the active ingredient in the ionic liquid and must further form microemulsions.

Hence, in the field of transdermal preparations, water-soluble active ingredients are difficult to be used and the types of usable active ingredients are limited. Further, even when the active ingredients can be prepared into transdermal preparations, their transdermal absorbability is not sufficient.

Thus, an object of the present invention is to provide a transdermal absorption enhancer that allows for easy production of transdermal preparations excellent in transdermal absorbability using various active ingredients.

Solution to Problem

The present inventors have conducted diligent studies in light of the present situation mentioned above and consequently completed the present invention by finding the fact that a transdermal preparation having an excellent transdermal absorbability of not only poorly soluble active ingredients but various water-soluble active ingredients (e.g., acyclovir, mizoribine, and methotrexate) can be obtained by using an equimolar salt of a combination of a particular basic functional group-containing compound and a particular acidic functional group-containing compound as a transdermal absorption enhancement aid or a transdermal absorption enhancer.

Specifically, the present invention is as follows:

[1] A transdermal absorption enhancement aid or a transdermal absorption enhancer comprising an equimolar salt of

a) a basic functional group-containing compound having a molecular weight of from 50 to 120 and a melting point of from 50 to 350°C, and
b) an acidic functional group-containing compound having a LogP from of -4 to 7.3.

[2] The transdermal absorption enhancement aid or the transdermal absorption enhancer according to [1] wherein the LogP of the acidic functional group-containing compound b) is from 2.5 to 7.3.

[3] The transdermal absorption enhancement aid or the transdermal absorption enhancer according to [1] or [2], wherein the acidic functional group-containing compound b) is one compound selected from the group consisting of an amino acid, a carboxylic acid, a hydroxy acid, a long-chain fatty acid having from 12 to 20 carbon atoms and optionally having a substituent, and a saccharic acid.

[4] The transdermal absorption enhancement aid or the transdermal absorption enhancer according to [3], wherein the acidic functional group-containing compound b) is one compound selected from the group consisting of histidine, glycolic acid, lactate, malonic acid, acetic acid, maleic acid, succinic acid, glutaric acid, benzoic acid, capric acid, oleic acid, linoleic acid, and isostearic acid.

[5] The transdermal absorption enhancement aid or the transdermal absorption enhancer according to any of [1] to [4], wherein the basic functional group-containing compound a) is one compound selected from the group consisting of choline or a derivative thereof, histamine or a derivative thereof, and guanidine or a derivative thereof.

[6] The transdermal absorption enhancement aid or the transdermal absorption enhancer according to [5], wherein the basic functional group-containing compound a) is one compound selected from the group consisting of choline, histamine, and guanidine.

[7] The transdermal absorption enhancement aid or the transdermal absorption enhancer according to [6], wherein the basic functional group-containing compound a) is one compound selected from choline, histamine, and guanidine, and the acidic functional group-containing compound b) is one compound selected from capric acid, oleic acid, linoleic acid, and isostearic acid.

[8] A transdermal preparation comprising an active ingredient and a transdermal absorption enhancement aid or a transdermal absorption enhancer according to any of [1] to [7].

[9] The transdermal preparation according to [8], wherein the active ingredient has a molecular weight of 10000 or smaller and has a LogP of 3.5 or lower.

[10] The transdermal preparation according to [8], wherein the active ingredient has a molecular weight of 10000 or smaller and has a LogP of 0.5 or lower.

[11] The transdermal preparation according to any of [8] to [10], wherein the active ingredient is not a salt.

[12] The transdermal preparation according to any of [8] to [11], wherein the active ingredient is a therapeutic drug for viral infection or an immunosuppressive agent.

Advantageous Effects of Invention

The transdermal absorption enhancer and the transdermal absorption enhancement aid of the present invention can achieve the favorable transdermal absorption of an active ingredient by merely mixing with the active ingredient (by merely preparing a composition comprising the active ingredient and the transdermal absorption enhancer of the present invention, or a composition comprising the active ingredient, a transdermal absorption enhancer other than the transdermal absorption enhancer of the present invention, and the transdermal absorption enhancement aid of the present invention), and furthermore, can be applied not only to poorly soluble active ingredients but to water-soluble active ingredients. Therefore, transdermal preparations excellent in transdermal absorbability can be easily produced using various active ingredients.

Brief Description of Drawings

[Figure 1] Figure 1 is a diagram showing the relationship between the transdermal penetration amount of acyclovir in the presence of the transdermal absorption enhancement aid of the present embodiment and the LogP of the transdermal absorption enhancer used in combination therewith.

[Figure 2] Figure 2 is a diagram and an expression showing a transdermal absorption model (Hatanaka model) known in the art.

[Figure 3] Figure 3 is a diagram showing change in MZR concentration in blood in using the transdermal preparations of Examples 59 and 60, and Comparative Example 26.

Description of Embodiments

Hereinafter, the mode for carrying out the present invention (hereinafter, referred to as the "present embodiment") will be described in detail. However, the present invention is not intended to be limited by the present embodiment, and various changes or modifications can be made therein without departing from the spirit of the present invention.

(1. Transdermal absorption enhancer and transdermal absorption enhancement aid)

In the present embodiment, the "transdermal absorption enhancer" refers to an agent that can improve the transdermal absorbability of an active ingredient by coexisting with the active ingredient. The "transdermal absorption enhancement aid" refers to an agent that is used in combination with a transdermal absorption enhancer and can increase the transdermal absorbability-improving effect of the transdermal absorption enhancer.

In the present embodiment, the transdermal absorption enhancer and the transdermal absorption enhancement aid each comprise an equimolar salt of a basic functional group-containing compound a) and an acidic functional group-containing compound b) as an active ingredient.

The basic functional group-containing compound a) has a molecular weight of from 50 to 120 and a melting point of from 50 to 350°C. The molecular weight of the basic functional group-containing compound a) is preferably from 60 to 115, more preferably from 60 to 105.

It was found from the studies of the present inventors that: the molecular weight of the basic functional group-containing compound a) is related to the affinity between the equimolar salt and an active ingredient, and the melting point of the basic functional group-containing compound a) is related to the affinity between the equimolar salt and the skin; and both of the molecular weight and the melting point of the basic functional group-containing compound a) need to be appropriately set for the transdermal penetration of the active ingredient. Specifically, the molecular weight needs to be from 50 to 120 and is preferably from 60 to 115, more preferably from 60 to 105. The melting point is from 50 to 350°C, preferably from 50 to 330°C, more preferably from 50 to 310°C.

The LogP (common logarithm of partition coefficient P between water and 1-octanol) of the acidic functional group-containing compound b) also needs to be from -4 to 7.3. The LogP is preferably from 0 to 7.3, more preferably from 2 to 7.3, particularly preferably from 2.5 to 7.3.

It was found from the studies of the present inventors that the LogP of the acidic functional group-containing compound b) is related to the affinity between the equimolar salt and the skin. However, its preferred range differs depending on the degree of hydration of the skin. The optimum LogP tends to be lower for the more hydrated skin. It was also found from the studies of the present inventors that if the LogP exceeds 7.3, the transdermal penetrability of an active ingredient is significantly reduced, regardless of the degree of hydration of the skin. This may be because the active ingredient cannot be distributed to the skin when the Log P is too large, though the mechanism underlying this is not limited.

The basic functional group-containing compound a) may be any compound as long as the compound contains a basic functional group and has a molecular weight and a melting point that fall within the ranges described above.

The LogP thereof is not limited and can be set to, for example, a value equivalent to or smaller than that of the acidic functional group-containing compound b).

Specific examples thereof include choline or a derivative thereof, histamine or a derivative thereof, and guanidine or a derivative thereof. Among them, a substance present *in vivo* (i.e., a substance ingestible into an organism, or a substance newly produced *in vivo* as a result of the *in vivo* catabolism or anabolism of the ingestible substance) is preferred from the viewpoint of safety. Such a substance is a constituent of a nucleic acid, a protein, a sugar, a lipid, a vitamin, a hormone, or the like, or is a substance involved in *in vivo* necessary synthesis and metabolic pathway, etc.

Among those described above, choline, histamine, and guanidine are particularly preferred from the viewpoint of the transdermal absorbability-enhancing effect on an active ingredient.

The acidic functional group-containing compound b) may be any compound as long as the compound contains a basic functional group and has a LogP of from -4 to 7.3.

Specific examples of the acidic functional group-containing compound b) include an amino acid, a carboxylic acid, a hydroxy acid, a long-chain fatty acid having from 12 to 20 carbon atoms and optionally having a substituent (wherein the from 12 to 20 carbon atoms mean the number of carbon atoms in the principal chain of the fatty acid), and a saccharic acid. A long-chain fatty acid having from 12 to 20 carbon atoms is preferred.

Among those described above, histidine, glycolic acid, lactate, malonic acid, acetic acid, maleic acid, succinic acid, glutaric acid, benzoic acid, capric acid, oleic acid, linoleic acid, and isostearic acid are particularly preferred from the viewpoint of the transdermal absorbability-enhancing effect.

In the present embodiment, the equimolar salt of the basic functional group-containing compound a) and the acidic functional group-containing compound b) is preferably a liquid at room temperature (23°) from the viewpoint of handling, but it is not necessarily required to be a liquid.

The transdermal absorption enhancer or the transdermal absorption enhancement aid may comprise, in addition to the equimolar salt described above, impurities derived from the basic functional group-containing compound a) or the acidic functional group-containing compound b) used, or an excess amount over the equal mole of the basic functional group-containing compound a) or the acidic functional group-containing compound b).

The combination of the acidic functional group of the acidic functional group-containing compound b) and the basic functional group of the basic functional group-containing compound a) in the equimolar salt is not limited and is preferably a combination in which the acid dissociation constant (pKa) of the acidic functional group in water is smaller than that of the basic functional group in water. The value of pKa of the acidic functional group is preferably from 4 to 6. The value of pKa of the basic functional group (if the functional group has two or more pKas, the higher value) is preferably from 8 to 15.

The basic functional group of the basic functional group-containing compound a) is not limited. Preferred examples of the basic functional group include a substituted or unsubstituted amino group (including substituted or unsubstituted ammonium salt) and an imidazole group (including imidazolium salt).

The basic functional group-containing compound a) is preferably a compound containing any of a quaternary ammonium group, an amino group, and an imidazole group. Examples thereof include choline, histamine, and guanidine.

The acidic functional group of the acidic functional group-containing compound b) is not limited. Preferred examples of the acidic functional group include a phosphoric acid group, a sulfonyl group, a carbonate group, and a carboxyl group.

Preferred examples of the acidic functional group-containing compound b) include an amino acid, a carboxylic acid, a hydroxy acid, a long-chain fatty acid having from 12 to 20 carbon atoms in the principal chain, a carboxylic acid other than the hydroxy acid and the long-chain fatty acid, and a saccharic acid, containing a carboxyl group.

The amino acid is not particularly limited and is preferably an $\alpha$-amino acid, more preferably glycine, glutamic acid, isoleucine, aspartic acid, or histidine.

The hydroxy acid is not particularly limited as long as the hydroxy acid is a carboxylic acid having a hydroxy group. The hydroxy acid is preferably glycolic acid, lactic acid, vanillylmandelic acid, malic acid, or citric acid.

The saccharic acid may be any of aldonic acid, aldaric acid, and uronic acid and is preferably glucuronic acid.

The long-chain fatty acid is not particularly limited as long as the number of carbon atoms in its principal chain is from 12 to 20. The long-chain fatty acid may have a substituent. Examples thereof include dodecanoic acid (lauric acid), myristic acid, palmitic acid, stearic acid, capric acid, oleic acid, linoleic acid, and isostearic acid. The long-chain fatty acid is preferably capric acid, oleic acid, linoleic acid, or isostearic acid.

The carboxylic acid other than the hydroxy acid and the long-chain fatty acid can also be used. Examples thereof include acetic acid, propionic acid, isobutyric acid, maleic acid, fumaric acid, malonic acid, succinic acid, glutaric acid, adipic acid, and benzoic acid.

In the present embodiment, the equimolar salt contained in the transdermal absorption enhancer or the transdermal absorption enhancement aid is particularly preferably an equimolar salt of one compound a) selected from the group consisting of choline, histamine, and guanidine and one compound b) selected from the group consisting of histidine, glycolic acid, lactic acid, malonic acid, acetic acid, maleic acid, succinic acid, glutaric acid, benzoic acid, capric acid, oleic acid, linoleic acid, and isostearic acid.

Specifically, the equimolar salt is choline-histidine salt, choline-glycolic acid salt, choline-lactic acid salt, choline-acetic acid salt, choline-maleic acid salt, choline-succinic acid salt, choline-glutaric acid salt, choline-benzoic acid salt, choline-capric acid salt, choline-oleic acid salt, choline-linoleic acid salt, choline-isostearic acid salt, guanidine-capric acid salt, guanidine-oleic acid salt, guanidine-linoleic acid salt, guanidine-isostearic acid salt, histamine-capric acid salt, histamine-oleic acid salt, histamine-linoleic acid salt, and histamine-isostearic acid salt. Particularly preferred examples thereof include choline-capric acid salt, choline-oleic acid salt, choline-linoleic acid salt, choline-isostearic acid salt, choline-succinic acid salt, choline-benzoic acid salt, guanidine-capric acid salt, guanidine-oleic acid salt, guanidine-linoleic acid salt, guanidine-isostearic acid salt, histamine-capric acid salt, histamine-oleic acid salt, histamine-linoleic acid salt, and histamine-isostearic acid salt.

(2. Active ingredient that can be used in transdermal preparation)

In the present embodiment, the transdermal preparation comprises at least the equimolar salt of the basic functional group-containing compound a) and the acidic functional group-containing compound b) described above, and an active ingredient.

The active ingredient is not limited. The transdermal absorption enhancer and the transdermal absorption enhancement aid of the present embodiment have a transdermal absorption-enhancing effect on both poorly soluble and water-soluble active ingredients. The transdermal absorption enhancer and the transdermal absorption enhancement aid of the present embodiment are particularly advantageous in producing a transdermal absorption-enhancing effect even on water-soluble active ingredients, which have been difficult to formulate into transdermal absorption preparations until now. The water-soluble active ingredient, as used herein, refers to an active ingredient having a solubility of 0.5 mg/mL or higher in water at 20°C. The solubility is preferably 10 mg/mL or higher, more preferably 30 mg/mL or higher, particularly preferably 100 mg/mL or higher.

Also, a water-soluble active ingredient having two or more acid dissociation constants (pKas) is preferred.

The molecular weight of the water-soluble active ingredient is preferably 10000 or smaller.

The water-soluble active ingredient having a common logarithm of partition coefficient P between water and 1-octanol (LogP) of 3.5 or lower is preferred, the LogP is more preferably lower than 2, and particularly preferably 0.5 or lower.

When the LogP is 0, the active ingredient is evenly distributed to water and 1-octanol. When the LogP is 3.5 or lower, the active ingredient has higher water solubility and lower transdermal absorbability. Therefore, the transdermal absorption enhancer and the transdermal absorption enhancement aid of the present embodiment produce more marked effects.

In the transdermal preparation of the present embodiment, the water-soluble active ingredient may be in a salt state or many be in a free state which is not a salt.

When the LogP is low, for example, the LogP is 3.5 or lower, it is preferred that the active ingredient is not a salt, i.e., it is preferred not to form a salt such as sulfate, hydrochloride, lactate, or phosphate.

The LogP is a value generally used as an index for hydrophilicity-hydrophobicity of a compound and is a logarithm of a partition coefficient actually measured by the shake-flask method ((1) water and 1-octanol are mixed for 24 hours or longer for saturation; (2) the mixture is placed, together with a substance to be assayed, in a flask to shake the flask; (3) phase separation is caused by centrifugation; and (4) the substance to be assayed contained in each phase is quantified) using 1-octanol/water (buffer solution of pH 7.4). The LogP can also be calculated with software "XLogP" (available from Shanghai Institute of Organic Chemistry, Chinese Academy of Sciences, http://WWW.sio-ccbg.ac.cn/software/xlogp3/), and the calculation results stored in database PubChem (http://pubchem.ncbi.nlm.nih.gov/#) can be used.

The transdermal absorption agent and the transdermal absorption enhancement aid of the present embodiment are effective for even water-soluble active ingredients and can be applied to a wide range of active ingredients, etc., for pharmaceutical products, cosmetics, quasi-drugs, and the like.

Examples of the water-soluble active ingredient for use in pharmaceutical products include antipyretic, analgesic, and anti-inflammatory agents (acetaminophen, LogP = 0.5, aspirin, LogP = 1.2, isopropylantipyrine, LogP = 1.7, etc.), hypnotic sedatives (chloral hydrate, LogP = 1, bromovalerylurea, LogP = 1.1, etc.), sleep-averting agents (caffeine, LogP = - 0.1, etc.), antivertigo agents, steroids (prednisolone, LogP = 1.6, methylprednisolone, LogP = 1.9, betamethasone, LogP = 1.9), analgesic agents for children, stomachics (hyoscyamine, LogP = 1.7, ethyl aminobenzoate, LogP = 1.9, etc.), antacids (famotidine, LogP = -0.6, ranitidine, LogP = 0.3, cimetidine, LogP = 0.4, lafutidine, LogP = 1.4, nizatidine, LogP = 1.6, omeprazole, LogP = 2.2, esomeprazole, LogP = 2.2, lansoprazole, LogP = 2.8, etc.), and digestive agents.

Other examples thereof include cardiotonic agents (dopamine, LogP = -1, methylxanthine, LogP = -0.7, isoprenaline, LogP = -0.6, amrinone, LogP = -0.2, milrinone, LogP = 0, digoxin, LogP = 1.3, denopamine, LogP = 1.8, methyldigoxin, LogP = 1.8, digitoxin, LogP = 2.3, vesnarinone, LogP = 2.1, docarpamine, LogP = 2.9, etc.), drugs for arrhythmia (ATP, LogP = -5.7, sotalol, LogP = 0.2, atenolol, LogP = 0.2, procainamide, LogP = 0.9, nifekalant, LogP = 1.1, bisoprolol, LogP = 1.9, mexiletine, LogP = 2.1, lidocaine, LogP = 2.3, pilsicainide, LogP = 2.7, etc.), hypotensive agents (lisinopril, LogP = -2.9, methyldopa, LogP = -1.9, imidapril, LogP = -0.7, enalapril, LogP = -0.1, captopril, LogP = 0.3, cilazapril, LogP = 0.6, perindopril, LogP = 0.9, temocapril, LogP = 1, quinapril, LogP = 1.2, benazepril, LogP = 1.3, terazosin, LogP = 1.4, urapidil, LogP = 1.5, clonidine, LogP = 1.6, delapril, LogP = 1.7, prazosin, LogP = 2, trandolapril, LogP = 2, guanfacine, LogP = 2, alacepril, LogP = 2.1, bunazosin, LogP = 2.2, nifedipine, LogP = 2.2, arotinolol, LogP = 2.3, doxazocin, LogP = 2.5, phentolamine, LogP = 2.6, tolazoline, LogP = 2.6, amlodipine, LogP = 3, bevantolol, LogP = 3), vasodilators (hydralazine, LogP = 0.7, loniten, LogP = 1.2, penthrite, LogP = 1.4, etc.), and diuretics (isosorbide, LogP = -1.4, acetazolamide, LogP = -0.3, hydrochlorothiazide, LogP = -0.1, meticrane, LogP = 0.5, trichlormethiazide, LogP = 0.6, chlorthalidone, LogP = 0.9, mefruside, LogP = 0.9, triamterene, LogP = 1, eplerenone, LogP = 1.4, furosemide, LogP = 2, torasemide, LogP = 2.7, etc.).

Other examples thereof include antiulcer agents (pirenzepine, LogP = 0.1, butylscopolamine bromide, LogP = 0.9, rebamipide, LogP = 2.4, etc.), intestinal regulators (guaiacol, LogP = 1.3, etc.), therapeutic drugs for osteoporosis (parathormon, LogP = -18.7, elcitonin, LogP = -13.3, alendronate, LogP = -6.5, risedronate, LogP = -3.5, etidronic acid, LogP = - 3.7, etc.), antirheumatic agents (mizoribine, LogP = -1.9, D-penicillamine, LogP = -1.8, methotrexate, LogP = -1.8, actarit, LogP = 0.4, epirizole, LogP = 1.3, leflunomide, LogP = 2.6, tacrolimus, LogP = 2.7), antitussive agents, antiasthmatic agents, antimicrobial agents, pollakiuria-ameliorating agents, revitalizers, and vitamins.

Examples of the water-soluble active ingredient for use in cosmetics and quasi-drugs include skin-lightening agents, antiaging agents, antioxidants, moisturizers, hair growers, cell activators, vitamins, and amino acids. Specific examples thereof include arbutin (LogP = -0.6), L-ascorbic acid (LogP = -1.6), hydroquinone (LogP = -0.6), glutathione (LogP = -4.5), placenta extracts, pantothenic acid (LogP = -1.1), tranexamic acid (LogP = - 2), kojic acid (LogP = -0.9), L-cysteine (LogP = -2.5), ellagic acid (LogP = 1.1), rucinol (LogP = 2.4), resorcin (LogP = 0.8), astaxanthin and derivatives thereof, rutin (LogP = -1.3), cholesterol and derivatives thereof, tryptophan (LogP = -1.1), histidine (LogP = -3.2), flavonoids such as quercetin (LogP = 1.5) and quercitrin (LogP = 0.9), catechin (LogP = 0.4) and derivative thereof, gallic acid (LogP = 0.7) and derivatives thereof, kinetin (LogP = 1), α-lipoic acid (LogP = 1.7), erythorbic acid (LogP = -1.6) and derivatives thereof, thiotaurine (LogP = -0.1), urea (LogP = -1.4), nicotine (LogP = 1.2) and derivatives thereof, nicotinic acid (LogP = 0.4), hydroxyproline (LogP = -3.3), serine (LogP = -3.1), glutamic acid (LogP = -3.7), arginine (LogP = -4.2), alanine (LogP = -3), minoxidil (LogP = 1.2), D-glucosamine (LogP = -2.8), N-acetyl-D-glucosamine (LogP = -1.7), hyaluronic acid (LogP = -7.4), raffinose (LogP = -5.8), azelaic acid (LogP = 1.6), γ-aminobutyric acid (LogP = -3.2), allantoin (LogP = -2.2), L-carnitine (LogP = -0.2), and biotin (LogP = 0.3).

(3. Transdermal absorption enhancer that can be used in combination with transdermal absorption enhancement aid of present embodiment)

In order to achieve a penetration through the stratum corneum of the skin, which works as an *in vivo* barrier, the transdermal absorption enhancement aid of the present embodiment can be used in combination with a transdermal absorption enhancer other than the transdermal absorption enhancer of the present embodiment (hereinafter, also simply referred to as an " the other transdermal absorption enhancer").

The other transdermal absorption enhancer is preferably a compound that penetrates through the skin and is in a liquid state at 23°C. The other transdermal absorption enhancer is not particularly limited as long as it is known in the art. For example, a compound described as an absorption enhancer in the Pharmaceutical Excipients Dictionary can be used, and a compound whose daily maximum dose is known is preferred.

Specific examples thereof include: polyethylene glycol, for example, PEG400; alcohols, for example, ethanol, isopropanol, benzyl alcohol, and octyldodecanol; polyhydric alcohols, for example, ethylene glycol, propylene glycol, butylene glycol, glycerin, and diethylene glycol monoethyl ether; fatty acids, for example, oleic acid, capric acid, and linoleic acid; esters, for example, ethyl acetate, isopropyl myristate (IPM), glycerin monooleate, glycerin tricaprylate (tricaprylin), and glyceryl tetrahydrofarnesylacetate; polyvalent carboxylic acid esters, for example, diethyl adipate, diisopropyl adipate, diethyl sebacate; α-hydroxy acids, for example, lactic acid and glycolic acid; surfactants, for example, sucrose oleic acid ester, sucrose lauric acid ester, polyoxyethylene-2-oleyl ether, and Tween 80; terpenes, for example, d-limonene, I-menthol, and peppermint oil; laurocapram; pyrrothiodecane; urea and derivatives thereof, for example, urea, 1,3-diphenylurea and cyclic urea derivatives; salicylic acid; thioglycolic acids, for example, calcium thioglycolate; pyrrolidones, for example, N-methyl-2-pyrrolidone and pyrrolidonecarboxylic acid; sulfoxides, for example, dimethyl sulfoxide and decyl methyl sulfoxide; alkyl-N,N-2-substituted aminoacetic acids, for example, dodecyl N,N-dimethylaminoacetate, dodecyl 2-methyl-2-(N,N-dimethylaminoacetate); dodecyl aminoacetate derivatives; cyclodextrins, for example, β-cyclodextrin and dimethylcyclodextrin; and liquid paraffin.

More preferred examples thereof include polyethylene glycol, glycerin, propylene glycol, 1,3-butylene glycol, ethanol, surfactants, fatty acids such as oleic acid and linoleic acid, tricaprylin, fatty acid esters such as IPM, polyvalent carboxylic acid esters such as diisopropyl adipate, and liquid paraffin.

The other transdermal absorption enhancer that is used with the transdermal absorption enhancement aid of the present embodiment preferably has a common logarithm of partition coefficient P between water and 1-octanol (LogP) of from -5 to 40. The LogP is more preferably from 7 to 40, further preferably from 7 to 20, particularly preferably from 8 to 20. Particularly preferred examples of the other transdermal absorption enhancer include IPM, tricaprylin, and octyldodecanol.

(4. Transdermal absorbability-improving effect)

The transdermal absorbability (transdermal penetrability)-improving effects of the transdermal absorption enhancer and the transdermal absorption enhancement aid according to the present embodiment on the active ingredient will be described below.

4.1 Effect as a transdermal absorption enhancement aid

First, the effect of the equimolar salt according to the present embodiment working as a transdermal absorption enhancement aid will be described.

The solubility of acyclovir (ACV) in a general transdermal absorption enhancer (the other transdermal absorption enhancer), and the influence of the transdermal absorption enhancement aid of the present embodiment on the transdermal penetration amount of ACV obtained by the Franz cell test were examined.

As a result, 1) it was confirmed that the transdermal absorption enhancement aid of the present embodiment improves the solubility of ACV in a general transdermal absorption enhancer by two times compared to the case without the addition of the transdermal absorption enhancement aid of the present embodiment (Table 1).

Particularly, what is remarkable is that a transdermal absorption enhancer, such as IPM, tricaprylin, or octyldodecanol, in which ACV is totally insoluble, becomes able to dissolve ACV, albeit in a trace amount. Although the reason for this is not clear, the transdermal absorption enhancement aid of the present embodiment probably acts on either of ACV or the other transdermal absorption enhancer, or both, to increase the solubility.

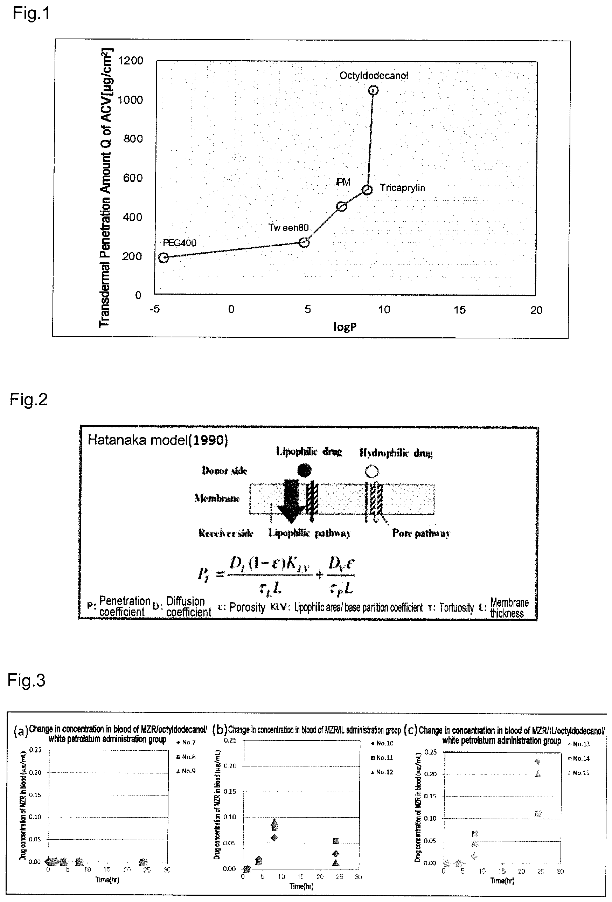

Further, 2) it was confirmed that, in the case of using the transdermal absorption enhancement aid of the present embodiment, the transdermal penetration amount of ACV is not proportional to the solubility of ACV in the other transdermal absorption enhancer, and the transdermal penetration amount increases as the LogP of the other transdermal absorption enhancer increases as long as ACV is dissolved even in a trace amount (see Figure 1; which is on the basis of data on Examples described in Tables 5 and 6 described later and shows the relationship between the LogP of the other transdermal absorption enhancer and the integrated transdermal penetration amount of ACV for 4 hours in the case of using choline-linoleic acid salt as the transdermal absorption enhancement aid of the present embodiment).

This suggests that the transdermal penetration amount of a water-soluble active ingredient can be increased by making the water-soluble active ingredient be dissolved in the other transdermal absorption enhancer having high LogP. In this respect, the transdermal absorption enhancement aid of the present embodiment that allows even a water-soluble active ingredient (which is totally insoluble in the other transdermal absorption enhancer) to be dissolved in the transdermal absorption enhancer seems to be very effective.

[Table 1]

<Table 1>

| The other transdermal absorption enhancer | | Solubility of ACV in the other transdermal absorption enhancer (mg/g) | | |
|---|---|---|---|---|
| Name | LogP | A: The transdermal absorption enhancement aid of the present embodiment (IL) was not added. | B: The IL was added. (The name of IL) | Improving effect (time) B/A×100 |
| PEG400 | -4.4 | 1.44 | 2.97 (Choline-linoleic acid) | 2.1 |
| Tween 80 | 4.8 | 0.23 | 0.99 (Choline-linoleic acid) | 4.3 |
| IPM | 7.2 | N.D. | 0.172 (Choline-linoleic acid) | Infinite |
| | | | 0.029 (Choline-benzoic acid) | Infinite |
| Tricaprylin | 8.9 | N.D. | 0.00009 (Choline-linoleic acid) | Infinite |
| Octyldodecanol | 9.2 | N.D. | 0.00026 (Choline-linoleic acid) | Infinite |

The Hatanaka model (Hatanaka T., Inuma M., Sugibayashi K. Morimoto Y., Chem. Pharm. Bull., 38, 3452-3459 (1990); and Morimoto Y., Hatanaka T., Sugibayashi K., Omiya H., J. Pharm. Pharmacol., 44, 634-639 (1992)) of Figure 2 is known as a model regarding transdermal absorption. According to this model, a lipophilic active ingredient is transported through a biomembrane (the first term of the right side in the expression of Figure 2), while a water-soluble active ingredient mainly penetrates through the skin via a pore present in the skin (the second term of the right side in the expression of Figure 2).

In this case, an apparent diffusion coefficient (DL) for the skin, and the value of skin/transdermal absorption enhancer partition coefficient (KLV) of the water-soluble active ingredient are dominant factors for determining the transdermal penetration amount of the drug. According to the conventional technical common sense, a highly lipophilic substance is easily distributed into the skin, but is low diffusible due to high affinity for the skin. Therefore, a transdermal absorption enhancer having LogP close to 1 offers the good balance between diffusion and distribution to the skin and is thus preferred.

However, as described above, it was confirmed that when the transdermal absorption enhancement aid of the present embodiment and the other transdermal absorption enhancer having large LogP are used in combination for a water-soluble active ingredient having small LogP, the transdermal penetration amount of the water-soluble active ingredient is large in spite of the fact that the water-soluble active ingredient is hardly dissolved in the transdermal absorption enhancer. This effect cannot be explained nor predicted from the conventional technical common sense, and may be brought about from a penetration mechanism that is different from the mechanism of the conventional transdermal penetration. Although the mechanism is not clear, it appears that either or both of the following take place: in the presence of the transdermal absorption enhancement aid of the present embodiment, ACV dissolved in the other transdermal absorption enhancer is well distributed into the skin without remaining in the other transdermal absorption enhancer; and/or the transdermal absorption enhancement aid of the present embodiment maintains its diffusibility without strengthening the affinity for the skin even when the LogP of the other transdermal absorption enhancer is large.

Non Patent Literature 1 states that in an experiment using Yucatan Micropig Skin as the skin, the transdermal penetration amount of ACV for 24 hours was 0.5 $\mu$g/cm$^2$ at maximum. By contrast, when the transdermal absorption enhancement aid of the present embodiment was used in combination with the other transdermal absorption enhancer (IPM, etc.), the transdermal penetration amount of ACV exceeded 1000 $\mu$g/cm$^2$ for 4 hours in some cases (e.g., Example 17 described later), though the skin used differed from that of Non Patent Literature 1.

Thus, the mechanism of the transdermal penetration of the water-soluble active ingredient brought about by use of the transdermal absorption enhancer of the present embodiment is presumably different from that is disclosed in Non Patent Literature 1.

Next, the effect of the transdermal absorption enhancement aid of the present embodiment was confirmed with an immunosuppressive agent mizoribine (MZR; LogP = -1.9, molecular weight: 259). MZR, as with ACV, is also a water-soluble active ingredient that has not been reported to transdermally penetrate by using a conventional transdermal absorption enhancer alone. MZR was mixed with the transdermal absorption enhancement aid of the present embodiment and the other transdermal absorption enhancer (IPM). Although some mixtures did not form a paste, the transdermal penetration of MZR was confirmed in all samples. Particularly, the transdermal penetration was enhanced when the mixture was in a paste state. MZR is incompatible with IPM, but the transdermal penetration was achieved. This suggests that the presence of the transdermal absorption enhancement aid of the present embodiment facilitates dissolving a portion of MZR in IPM and MZR, as with ACV, can transdermally penetrate by dissolving in the other transdermal absorption enhancer.

As described above, in the present embodiment, when the equimolar salt is used as a transdermal absorption enhancement aid in combination with the other transdermal absorption enhancer, the transdermal absorbability of an active ingredient is thought to be improved by increasing the solubility of the active ingredient in the other transdermal absorption enhancer, though the mechanism underlying this is not limited.

4.2 Effect as a transdermal absorption enhancer

Next, the effect of the equimolar salt according to the present embodiment working as a transdermal absorption enhancer will be described.

The solubility of ACV in the transdermal absorption enhancer of the present embodiment was examined. As a result, the transdermal absorption enhancer of the present embodiment was confirmed to be able to dissolve a portion or the whole of ACV.

It was further confirmed that MZR exhibits favorable solubility in the transdermal absorption enhancer of the present embodiment. When the equimolar salt of the present embodiment was used as a transdermal absorption enhancer, the transdermal penetration amount of the active ingredient was higher than the value when the equimolar salt was used as a transdermal absorption enhancement aid with the other transdermal absorption enhancer such as IPM.

It is thought that the water-soluble active ingredient, a portion or the whole of which has been dissolved in the transdermal absorption enhancer of the present embodiment, diffuses into the skin while interacting with the transdermal absorption enhancer having affinity for the skin, and can therefore penetrate through the skin although it is water-soluble.

Patent Literatures 2 to 4 disclose transdermal preparations of various poorly soluble drugs. These poorly soluble drugs, however, differ from water-soluble active ingredients in molecular weight, water solubility, and LogP, etc of the drug.

Since the stratum corneum of the skin is lipophilic, water-soluble active ingredients have limits in their transdermal penetration (transdermal absorption). The transdermal absorption enhancer of the present embodiment raises the probability of the transdermal penetration of water-soluble active ingredients.

(5. Transdermal preparation)

In the present embodiment, the transdermal preparation comprises at least an equimolar salt of the basic functional group-containing compound a) and the acidic functional group-containing compound b) described above, and an active ingredient.

In the present embodiment, the transdermal preparation particularly preferably comprises a water-soluble active ingredient and the equimolar salt wherein the compound a) is a compound selected from the group consisting of choline or a derivative thereof, histamine or a derivative thereof, and guanidine or a derivative thereof, and the compound b) is a compound selected from the group consisting of an amino acid, a carboxylic acid, a hydroxy acid, a long-chain fatty acid having 10 to 20 carbon atoms, and a saccharic acid. More preferably, the molecular weight of the water-soluble active ingredient is 10000 or smaller.

In the present embodiment, the content of the active ingredient in the transdermal preparation is not limited. However, in order not to run out of the active ingredient to penetrate the skin after the transdermal penetration of the active ingredient dissolved in the transdermal preparation, it is preferred that the active ingredient is present in an excess amount over its solubility in the transdermal absorption enhancer (the transdermal absorption enhancer of the present embodiment or the other transdermal absorption enhancer). The undissolved active ingredient present is sequentially dissolved in the transdermal absorption enhancer and then transdermally penetrate. By repeating this cycle, the total transdermal penetration amount of the active ingredient will increase.

In the present embodiment, the transdermal preparation refers to a preparation that allows the active ingredient to be absorbed through the skin of humans or other animals (transdermally absorbed), and may be in a form such as a patch preparation, a cataplasm, an emulsion, a cream, a lotion, an essence, a mousse, a spray, a gel, or an oil, which allows the active ingredient to be transdermally absorbed by applying or pasting etc, in the use application of pharmaceutical products, cosmetics, and quasi-drugs.

In the case of a liniment, the transdermal absorption enhancer of the present embodiment contained therein can also be expected to produce a effect of improving the spreadability of the transdermal preparation to facilitate application to the skin.

In the present embodiment, the transdermal preparation is just required to contain a water-soluble active ingredient and the transdermal absorption enhancer or the transdermal absorption enhancement aid of the present embodiment. The content of the transdermal absorption enhancer or the transdermal absorption enhancement aid of the present embodiment is not limited. The content of the transdermal absorption enhancer of the present embodiment or the transdermal absorption enhancement aid of the present embodiment is preferably from 1 to 99.9% by mass with respect to the mass of the transdermal preparation.

When the content of the transdermal absorption enhancer of the present embodiment or the transdermal absorption enhancement aid of the present embodiment is 1% by mass or larger, the transdermal absorbability-improving effect is sufficiently produced. More preferably, the content is equimolar or larger with respect to the water-soluble active ingredient and it can be increased or decreased in light of the degree of the effect. On the other hand, when the content of the transdermal absorption enhancer or the transdermal absorption enhancement aid of the present embodiment is 99.9% by mass or smaller, the adequate efficacy of the active ingredient can be obtained.

In the present embodiment, the transdermal preparation may optionally comprise the other transdermal absorption enhancer described above, in addition to the transdermal absorption enhancer of the present embodiment or the transdermal absorption enhancement aid of the present embodiment. The amount of the other transdermal absorption enhancer is preferably from 0.01 to 10 times the mass of the water-soluble active ingredient.

The transdermal preparation may optionally further contain other additives including polyhydric alcohols, oils such as liquid paraffin, squalane, plant oil, higher fatty acid, and higher alcohol, organic acids such as citric acid and lactic acid, surfactants, pigments, dyes, antiseptics, resins, pH adjusters, antioxidants, ultraviolet absorbers, chelating agents, thickeners, moisturizers, alcohols, water, fragrances, etc.

In the present embodiment, it is preferred that the transdermal preparation should comprise no surfactant. The surfactant causes strong dermal irritation, and surfactant-free formulation is thus desirable.

When the transdermal preparation is an adhesive skin patch (patch preparation) which allows the active ingredient to be transdermally absorbed by pasting, etc., this preparation can be constituted such that a layer comprising the transdermal preparation is laminated on a support. In this case, a pressure-sensitive adhesive layer may be disposed on the layer comprising the transdermal preparation, or a pressure-sensitive adhesive may be added in the layer comprising the transdermal preparation. Examples of the pressure-sensitive adhesive include acrylic pressure-sensitive adhesives, natural rubber pressure-sensitive adhesives, synthetic rubber pressure-sensitive adhesives, silicone pressure-sensitive adhesives, vinyl ester pressure-sensitive adhesives, and vinyl ether pressure-sensitive adhesives. A pressure-sensitive adhesive whose dermal irritation, skin contact, and the like are easily-controllable, is preferred. These pressure-sensitive adhesives may each be used alone or may be used in combination of two or more. Alternatively, two or more pressure-sensitive adhesives may be laminated. A softening agent, a filler, an antioxidant, and the like may further be contained.

Examples of the synthetic rubber pressure-sensitive adhesives include polyisobutylene, polyisoprene, styrene-butadiene rubber, styrene-isoprene-styrene copolymers, styrene-butadiene-styrene block copolymers, styrene-ethylene-butadiene rubber-styrene block copolymers, polybutene, butyl rubber, and silicon rubber. Examples of the acrylic pressure-sensitive adhesives include acrylic acid alkyl ester and polymethacrylic acid alkyl ester.

Examples

Hereinafter, the present invention will be described in detail by way of Examples. However, the scope of the present invention is not intended to be limited by these Examples.

(Production Examples 1 to 37) An organic base (Choline hydroxide used as a starting material for choline (molecular weight: 104.17, melting point: 305°C) serving as a basic functional group-containing compound a) or guanidine (molecular weight: 59.07, melting point: 50°C) serving as a basic functional group-containing compound a)) as described in Table 2 was dissolved at a concentration of 0.15 to 0.375 mol/L in water and/or ethanol to obtain a base solution. Similarly, an organic acid (acidic functional group-containing compound b) (for LogP, see Table 19 described later)) was dissolved at a concentration of 0.15 to 0.375 mol/L in water and/or ethanol to obtain an acid solution. 50 to 60 mL (100- to 1000-mL eggplant-shaped flask was used) of the acid solution was mixed with the base solution in equimolar amounts of the basic functional group-containing compound a) and the acidic functional group-containing compound b) with stirring in an ice bath or, if necessary, at room temperature (20 to 25°C), to prepare a solution of an equimolar salt of the basic functional group-containing compound and the acidic functional group compound. Then, ethanol and water were removed by azeotropy using an evaporator, and the residue was washed three times with 75 mL of ether. After separation of the ether phase, the obtained residue was placed in a sample tube and dried at room temperature for 1 hour, at 40°C for 3 hours, and at 80°C for 4 hours in a vacuum dryer to obtain the equimolar salt of the basic functional group-containing compound a) and the acidic functional group compound b). The state of the obtained equimolar salt, etc., are shown in Table 2.

[Table 2]

<Table 2>

| Production Example | Organic base | Organic acid | | State | Odor<br>× Strong<br>Δ Weak<br>○ Favorable |
|---|---|---|---|---|---|
| 1 | Choline hydroxide<br><br>(LogP = -0.4(choline)) | Amino acid | Glycine (LogP=-3.21) | Liquid | × |
| 2 | | | Cysteine | Paste | × |
| 3 | | | Glutamic acid (LogP=-3.69) | Solid to starch syrup-like | ○ No odor |
| 4 | | | L-Isoleucine (LogP=-3.69) | Paste | × |
| 5 | | | DL-Aspartic acid | Solid | Δ |
| 6 | | | L-Aspartic acid | Liquid | × |
| 7 | | | L-Histidine | Starch syrup-like | × |
| 8 | | Hydroxy acid | Glycolic acid | Liquid | × |
| 9 | | | DL-Lactic acid | Liquid | Δ |
| 10 | | | Vanillylmandelic acid | Solid to starch syrup-like | ○ (Vanilla-like) |
| 11 | | | L-Malic acid | Deliquescent solid | Δ |
| 12 | | | DL-Malic acid | Solid | Δ |
| 13 | | | Citric acid | Starch syrup-like | Δ |
| 14 | | Monocarboxylic acid | Acetic acid | Deliquescent solid | × |
| 15 | | | Propionic acid | Liquid | × |
| 16 | | | Isobutyric acid | Liquid | × |

(continued)

| Production Example | Organic base | Organic acid | | State | Odor<br>× Strong<br>△ Weak<br>○ Favorable |
|---|---|---|---|---|---|
| 17 | | Dicarboxylic acid | Maleic acid ( LogP=-0.5) | Liquid | ○ No odor |
| 18 | | | Fumaric acid | Solid | × |
| 19 | | | Malonic acid | Liquid | × |
| 20 | | | Succinic acid (LogP=-0.6) | Solid | △ |
| 21 | | | Glutaric acid (LogP=-0.3) | Liquid | × |
| 22 | | | Adipic acid | Deliquescent solid | △ |
| 23 | | Aromatic carboxylic acid | Benzoic acid (LogP=1.9) | Liquid | ○ (Honey-like) |
| 24 | | Sugar | D-Glucuronic acid | Liquid | ○ (Aroma) |
| 25 | | Long-chain fatty acid | Dodecanoic acid | Solid | △ |
| 26 | | | Myristic acid | Solid | △ (Butter-like) |
| 27 | | | Palmitic acid | Solid | △ (Butter-like) |
| 28 | | | Stearic acid | Solid | ○ No odor |
| 29 | | | Oleic acid | Gel | × |
| 30 | | | Linoleic acid (LopP=7.05) | Gel | △ |
| 31 | Guanidine | Amino acid | Glycine | Solid | × |
| 32 | | Hydroxy acid | Vanillylmandelic acid | Solid | ○ (Vanilla-like) |
| 33 | | Dicarboxylic acid | Maleic acid | Solid | ○ No odor |
| 34 | | Aromatic carboxylic acid | Benzoic acid | Solid | ○ No odor |
| 35 | | Sugar | D-Glucuronic acid | Solid | ○ No odor |
| 36 | | Long-chain fatty acid | Oleic acid | Gel | △ |
| 37 | | | Linoleic acid | Gummy | △ |

I. Evaluation of solubility of active ingredient

(Examples 1 to 13)

50 mg of ACV (the water-soluble active ingredient), 3000 mg of IPM (the other transdermal absorption enhancer), and the equimolar salt (the transdermal absorption enhancement aid of the present embodiment) produced in each of Production Examples 7, 14, 17, 19, 23, 25 to 30, 36, and 37 (in an equimolar amount with respect to ACV) were placed in a test tube, dispersed for 1 minute with a mixer (AUTOMATIC LAB-MIXER, model HM-10, the number of rotations: 2600 rpm, manufactured by AS ONE Corp.), and then ultrasonicated for 1 hour (an ultrasonic washing apparatus UC-6200 manufactured by Sharp Corp. was used for the ultrasonication; highfrequency output: 600 W, 40 kHz, ferrite oscillator, dial: largest). The dispersion thus treated was centrifuged (5200 G, 3 min), and the supernatant (main component: the other transdermal absorption enhancer) was filtered through a PTFE filter (pore size: 0.45 μm), followed by analysis on the amount of the drug by HPLC. Since the direct measurement of the supernatant resulted in column clogging, an HPLC eluent in the same amount as that of the supernatant was added to the supernatant and the eluent (lower layer) was collected and conducted the HPLC analysis.

The solubility of ACV in IPM in the presence of the transdermal absorption enhancement aid of the present embodiment is shown in Table 3. As shown in Comparative Example 1 described later, ACV is hardly dissolved in IPM without the transdermal absorption enhancement aid of the present embodiment.

(HPLC analysis conditions for ACV)

High-performance liquid chromatographic apparatus (model: LC-20AD, manufactured by Shimadzu Corp.)

UV detector (model: SPD-20A, manufactured by Shimadzu Corp.)

Column oven (model: CO705, manufactured by GL Sciences Inc.)

Colum: COSMOSIL 5C18-PAQ, column diameter: 4.6 mm I.D. × 150 mm, manufactured by Nacalai Tesque, Inc.

Eluent; 4% aqueous acetonitrile solution + 0.1% acetic acid

Temperature; 40°C, flow rate: 0.6 mL/min, detection wavelength: 254 nm

Retention time; 4.5-7.0 min

[Table 3]

<Table 3>

| Example | Transdermal absorption enhancement aid | ACV solubility in IPM in presence of transdermal absorption enhancement aid [μg/mL-IPM] (ACV is hardly dissolved in IPM) |
|---|---|---|
| 1 | Production Example 7 (Choline-histidine) | 2.4 |
| 2 | Production Example 14 (Choline-acetic acid) | 20.2 |
| 3 | Production Example 17 (Choline-maleic acid) | 11.5 |
| 4 | Production Example 19 (Choline-malonic acid) | 1.5 |
| 5 | Production Example 23 (Choline-benzoic acid) | 24.8 |
| 6 | Production Example 25 (Choline-dodecanoic acid) | 0.2 |
| 7 | Production Example 26 (Choline-myristic acid) | 1.8 |
| 8 | Production Example 27 (Choline-palmitic acid) | 1.4 |
| 9 | Production Example 28 (Choline-stearic acid) | 0.4 |
| 10 | Production Example 29 (Choline-oleic acid) | 13.7 |

(continued)

| Example | Transdermal absorption enhancement aid | ACV solubility in IPM in presence of transdermal absorption enhancement aid [μg/mL-IPM] (ACV is hardly dissolved in IPM) |
|---|---|---|
| 11 | Production Example 30 (Choline-linoleic acid) | 146.4 |
| 12 | Production Example 36 (Guanidine-Oleic acid) | 7.4 |
| 13 | Production Example 37 (Guanidine-Linoleic acid) | 10.8 |

(Examples 14 to 17 and Comparative Example 6)

50 mg of ACV, 3000 mg of each transdermal absorption enhancer (the other transdermal absorption enhancer) (any one of PEG400, Tween 80, tricaprylin, or octyldodecanol), and the equimolar amount of ACV of the equimolar salt produced in each of Production Examples 23 and 30 (the transdermal absorption enhancement aid of the present embodiment) or TIPA-capric acid described in Patent Literature 4 were placed in a test tube, dispersed for 1 minute with a mixer, and then ultrasonicated for 1 hour. The dispersion was centrifuged (5200 G, 3 min), and the supernatant (main component: the transdermal absorption enhancer) was filtered through a PTFE filter (pore size: 0.45 μm), followed by analysis on the amount of the drug by HPLC. In the case of IPM, since the direct measurement of the supernatant resulted in column clogging, an HPLC eluent in the same amount as that of the supernatant was added to the supernatant and the eluent (lower layer) was collected and conducted the HPLC analysis. The solubility of ACV in each transdermal absorption enhancer in the presence of the transdermal absorption enhancement aid of the present embodiment is shown in Table 4.

(Comparative Examples 1 to 5)

50 mg of ACV and 3000 mg of each transdermal absorption enhancer (the other transdermal absorption enhancer) (any one of IPM, PEG400, Tween 80, tricaprylin, or octyldodecanol) were placed in a test tube, dispersed for 1 minute with a mixer, and then ultrasonicated for 1 hour. The dispersion was centrifuged (5200 G, 3 min), and the supernatant (main component: the transdermal absorption enhancer) was filtered through a PTFE filter (pore size: 0.45 μm), followed by analysis on the amount of the drug by HPLC. In the case of IPM, since the direct measurement of the supernatant resulted in column clogging, an HPLC eluent in the same amount as that of the supernatant was added to the supernatant and the eluent (lower layer) was collected and conducted the HPLC analysis. The solubility of ACV in each transdermal absorption enhancer is shown in Table 4.

The transdermal absorption enhancement aid of the present embodiment improved the solubility of ACV in all of the transdermal absorption enhancers (the other transdermal absorption enhancers). Particularly, IPM, tricaprylin (medium-chain fatty acid), and octyldodecanol, in which ACV was totally insoluble, became able to dissolve ACV.

[Table 4]

<Table 4>

| Example | Transdermal absorption enhancer | | Transdermal absorption enhancement aid of present invention | ACV solubility in transdermal absorption enhancer in presence of transdermal absorption enhancement aid of present invention [mg/g-IPM] (ACV is hardly dissolved in IPM) |
|---|---|---|---|---|
| | Type | LogP | | |
| Comparative Example 2 | PEG400 | -4.4 | None | 1.44 |
| Example 14 | | | Production Example 30 | 2.97 |

(continued)

| Example | Transdermal absorption enhancer | | Transdermal absorption enhancement aid of present invention | ACV solubility in transdermal absorption enhancer in presence of transdermal absorption enhancement aid of present invention [mg/g-IPM] (ACV is hardly dissolved in IPM) |
|---|---|---|---|---|
| | Type | LogP | | |
| Comparative Example 3 | TWEEN80 | 4.8 | None | 0.23 |
| Example 15 | | | Production Example 30 | 0.99 |
| Comparative Example 1 | IPM | 7.2 | None | N.D. (not detected) |
| Example 5 | | | Production Example 23 | 0.029 |
| Comparative Example 1 | IPM | 7.2 | None | N.D. (not detected) |
| Example 11 | | | Production Example 30 | 0.172 |
| Comparative Example 4 | Tricaprylin (medium-chain fatty acid) | 8.9 | None | N.D. (not detected) |
| Example 16 | | | Production Example 30 | 0.00009 |
| Comparative Example 5 | Octyldodecanol | 9.2 | None | N.D. (not detected) |
| Comparative Example 6 | | | TIPA-capric acid | 0.00029 |
| Example 17 | | | Production Example 30 | 0.00026 |

II. Evaluation of transdermal absorbability

II-1 The case using artificial skin (Franz cell test)

(Examples 5, 11, and 14 to 17 and Comparative Examples 1 to 6)

The dispersions prepared in the same way as in Examples 5, 11, and 14 to 17 and Comparative Examples 1 to 6 were used, and the Franz cell test (apparatus name: Microette Plus 60-301-106, Hanson Research Corp., vertical diffusion cell: 7 mL, model 58-001-457, artificial skin: TESTSKIN TMLSE-003 manufactured by Toyobo Co., Ltd., receptor solution: PBS(-) manufactured by Wako Pure Chemical Industries, Ltd., temperature (set value): 32.5°C, effective area: $0.75 \text{ cm} \times 1.75 \text{ cm} \times 3.14 = 1.766 \text{ cm}^2$, the time of sampling: 0.5, 1, 1.5, 2, 4, 8, 12, 18, and 24 hours, measured at n = 2; the measurement was performed in a batch manner, and 1.5 ml of a sampled solution was obtained by ejection with 1.5 ml of a buffer solution at the time of each sampling run; this operation was performed twice, and the first solution was discarded as a rinse solution) was conducted. The transdermal penetration amount of the drug was determined according to the following expression:

$$\text{Transdermal penetration amount} = (C_nV + \Sigma(I = 1, n - 1)C_iS) / A$$

$C_n$: drug concentration [$\mu$g/mL] of the sample taken at the $n^{th}$ sampling
V: Franz cell volume [mL]
$\Sigma(I = 1, n - 1)C_i$: total of the drug concentration from the first sampling to the $(n - 1)^{th}$ sampling (n is the last run)[$\mu$g/mL]

S: volume of the sampled solution [mL]
A: effective area of the membrane [cm$^2$]

The results are shown in Tables 5 and 6. In the tables, "Penetration Amount of ACV" at each Time [h] represents the penetration amount of the drug from 0 (immediately after the start of the test) to the sampling time [h]. For example, "penetration amount of ACV" at Time [h] = "0.5" represents the "penetration amount of from 0 to 0.5 hours", "penetration amount of ACV" at "1" represents the "penetration amount of from 0 to 1 hour", and "penetration amount of ACV" at "24" represents the " penetration amount of from 0 to 24 hours".

In every case of using transdermal absorption enhancers, the addition of the transdermal absorption enhancement aid of the present embodiment increased the penetration amount of ACV. Also, the penetration amount of ACV increased as the LogP of the transdermal absorption enhancer increased, i.e., as the hydrophobicity became stronger.

[Table 5]

<Table 5>

| | Transdermal penetration amount of ACV [μg/cm$^2$] | | | |
|---|---|---|---|---|
| Time [h] | Comparative Example 1 (IPM alone) | Example 5 (IPM + choline-benzoic acid) | Example 11 (IPM + choline-linoleic acid) | Comparative Example 6 (Octyldodecanol + TIPA-capric acid) |
| 0.5 | 26.6 | 25.4 | 42.2 | 5 |
| 1 | 28.0 | 47.6 | 108.0 | 8 |
| 1.5 | 30.4 | 78.8 | 171.3 | 11 |
| 2 | 33.8 | 101.4 | 225.0 | 13 |
| 4 | 48.4 | 194.8 | 456.7 | 30 |
| 8 | 73.1 | 430.7 | 783.6 | 65 |
| 12 | 91.6 | 564.5 | 844.7 | 91 |
| 18 | 108.7 | 929.7 | 906.4 | 127 |
| 24 | 145.9 | 1139.9 | 954.0 | 154 |

[Table 6]

<Table 6>

| Time [h] | Comparative Example 2 (PEG alone) | Example 14 (PEG + choline-linoleic acid) | Comparative Example 3 (Tween 80 alone) | Example 15 (Tween 80 + choline-linoleic acid) | Comparative Example 4 (Tricaprylin alone) | Example 16 (Tricaprylin + choline-linoleic acid) | Comparative Example 5 (Ooctyl-dodecanol alone) | Example 17 (Octyldodeca -nol + choline-linoleic acid) |
|---|---|---|---|---|---|---|---|---|
| | Transdermal penetration amount of MZR [$\mu$g/cm$^2$] | | | | | | | |
| 0.5 | 26.5 | 18.6 | 23.3 | 30.3 | 8.2 | 144.2 | 9.3 | 148.3 |
| 1 | 38.5 | 33.3 | 43.8 | 56.1 | 6.3 | 217.6 | 5.2 | 375.2 |
| 1.5 | 47.4 | 63.2 | 56.5 | 77.3 | 7.1 | 274.7 | 6.5 | 602.5 |
| 2 | 56.8 | 79.5 | 71.7 | 108.4 | 8.3 | 318.0 | 8 | 670.8 |
| 4 | 103.7 | 190.8 | 181.3 | 273.5 | 18.2 | 543.2 | 17.9 | 1054.0 |
| 8 | 211.7 | 364.4 | 323.1 | 473.2 | 39.4 | 646.3 | 41.7 | 1329.5 |
| 12 | 252.8 | 559.2 | 473.7 | 641.5 | 70.8 | 607.5 | 70.3 | 1395.4 |
| 18 | 368.1 | 782.7 | 736.1 | 924.6 | 98.2 | 580.0 | 99.2 | 1469.7 |
| 24 | 467.4 | 927.8 | 1023.1 | 1120.1 | 101.4 | 555.4 | 112.3 | 1545.3 |

(Examples 18 to 23 and Comparative Examples 7 to 8)

25 mg of MZR, 3000 mg of each transdermal absorption enhancer (the other transdermal absorption enhancer) (any one of IPM, tricaprylin, or propylene glycol), and any one of the transdermal absorption enhancement aid of the present embodiment (any one equimolar salt of choline-succinic acid or choline-glutaric acid prepared in the same way as in Production Examples 23, 30), benzoic acid, or an equimolar salt of triisopropanolamine and capric acid (transdermal absorption enhancement aid described in Patent Literature 4) (added in an equimolar amount with respect to MZR) were placed in a test tube, dispersed for 1 minute with a mixer, and then ultrasonicated for 1 hour.

(HPLC analysis conditions for MZR)

High-performance liquid chromatographic apparatus (model: LC-20AD, manufactured by Shimadzu Corp.)
UV detector (model: SPD-20A, manufactured by Shimadzu Corp.)
Column oven (model: CO705, manufactured by GL Sciences Inc.)
Colum: COSMOSIL 5C18-PAQ, column diameter: 4.6 mm I.D. $\times$ 150 mm, manufactured by Nacalai Tesque, Inc.
Eluent: 0.067% aqueous phosphoric acid solution
Temperature: 30°C, flow rate: 0.5 mL/min, detection wavelength: 280 nm
Retention time; 3.0-4.0 min

Using the prepared dispersion or drug mixture partially forming a paste-like consistency, the Franz cell test (artificial skin: TESTSKIN TMLSE-003 manufactured by Toyobo Co., Ltd., receptor solution: phosphate buffer solution-saline, temperature (set value): 32.5°C, effective area: 0.75 cm $\times$ 1.75 cm $\times$ 3.14 = 1.766 cm$^2$, the time of sampling: 0.5, 1, 1.5, 2, 4, 8, 12, 18, and 24 hours, measured at n = 2 for each sample; the measurement was performed in a batch manner, and 1.5 ml of a sampled solution was obtained by ejection with 1.5 ml of a buffer solution at the time of each sampling run; this operation was performed twice, and the first solution was discarded as a rinse solution) was conducted. The transdermal penetration amount was determined. The results are shown in Table 7.

MZR hardly transdermally penetrate by the combination of IPM and benzoic acid alone (Comparative Example 7) and the combination of IPM and the equimolar salt of triisopropanolamine and capric acid (transdermal absorption enhancement aid described in Patent Literature 4) (Comparative Example 8), whereas the preparations of Examples 18 to 23, in which the transdermal absorption enhancement aid of the present embodiment were added, have increased transdermal penetration amount of MZR. The preparations of Examples 18, 20, and 21, in which the drug (active ingredient) partially formed a paste-like consistency, had larger transdermal penetration amounts of the drug than that of the preparation of Example 19, in which the drug was not in a paste state. It is thought that, by forming a paste with the transdermal absorption enhancement aid of the present embodiment, the solubility of drug increased. The penetration amounts also increased by the addition of the transdermal absorption enhancement aid of the present embodiment, when the transdermal absorption enhancer was tricaprylin or propylene glycol.. In the case where the drug is soluble in the transdermal absorption enhancement aid of the present embodiment, the optimum value of the LogP of the transdermal absorption enhancer is thought to depend on the easiness to distribute to the skin, etc.

[Table 7]

<Table 7>

| Time [h] | Transdermal penetration amount of MZR [$\mu$g/cm$^2$] | | | | | | | |
|---|---|---|---|---|---|---|---|---|
| | Comparative Example 7 (IPM + benzoic acid) | Comparative Example 8 (IPM + triisopropanolamine-capric acid) | Example 18 (IPM + choline-benzoic acid) | Example 19 (IPM + choline-linoleic acid) | Example 20 (IPM + choline-succinic acid) | Example 21 (IPM + choline-glutaric acid) | Example 22 (Tricaprylin + choline-benzoic acid) | Example 23 (Propylene glycol + choline-benzoic acid) |
| State of preparation | Drug was dispersed (without forming a paste) | Drug was dispersed (without forming a paste) | Drug partially formed a paste | Drug was dispersed (without forming a paste) | Drug partially formed a paste | Drug partially formed a paste | Drug was dispersed (without forming a paste) | Drug was dispersed (without forming a paste) |
| 0.5 | 9.5 | 12.2 | 17.1 | 139.5 | 817.6 | 0.0 | 0.0 | 19.8 |
| 1 | 11.5 | 17.7 | 19.2 | 175.5 | 925.0 | 0.1 | 1.9 | 66.5 |
| 1.5 | 13.1 | 12.9 | 23.0 | 194.8 | 676.5 | 0.3 | 6.0 | 66.1 |
| 2 | 14.9 | 8.1 | 41.6 | 210.1 | 633.5 | 0.4 | 12.0 | 77.7 |
| 4 | 32.7 | 9.8 | 173.6 | 267.2 | 621.9 | 2.5 | 63.8 | 190.3 |
| 8 | 60.2 | 14.8 | 776.1 | 361.2 | 724.0 | 15.4 | n.d. | 319.0 |
| 12 | n.d | 18.2 | 1390.4 | 422.5 | n.d. | 197.3 | n.d. | n.d. |
| 18 | n.d. | 22.4 | 3008.0 | 580.2 | n.d. | 612.7 | n.d. | n.d |
| 24 | 158.2 | 22.1 | 4444.4 | 684.4 | 602.8 | 770.1 | 622.5 | 289.3 |
| n.d.; not measured | | | | | | | | |

(Examples 24 to 26)

25 mg of MZR, 0, 6.8, 100, or 3000 mg of the transdermal absorption enhancer (IPM) (the other transdermal absorption enhancer), and the transdermal absorption enhancement aid of the present embodiment (Production Example 23) were placed in a test tube, dispersed for 1 minute with a mixer, and then ultrasonicated for 1 hour. Using the prepared dispersion or drug mixture partially forming a paste-like consistency, the Franz cell test (artificial skin: TESTSKIN TMLSE-003 manufactured by Toyobo Co., Ltd., receptor solution: phosphate buffer solution-saline, temperature (set value): 32.5°C, effective area: 0.75 cm $\times$ 1.75 cm $\times$ 3.14 = 1.766 cm$^2$, the time of sampling: 0.5, 1, 1.5, 2, 4, 8, 12, 18, and 24 hours, measured at n = 2 for each sample; the measurement was performed in a batch manner, and 1.5 ml of a sampled solution was obtained by ejection with 1.5 ml of a buffer solution at the time of each sampling run; this operation was performed twice, and the first solution was discarded as a rinse solution) was conducted to determine the transdermal penetration amount of the drug. The results are shown in Table 8.

[Table 8]

<Table 8>

| | | Example 18 | Example 24 | Example 25 | Example 26 |
|---|---|---|---|---|---|
| | MZR | 25mg | 25mg | 25mg | 25mg |
| | IPM | 3000mg | 0mg | 6.8mg | 100mg |
| | Choline-benzoic acid | Equimolar | 200mg | 200mg | 200mg |
| Time [h] | State of preparation | Drug was partially formed a paste | Completely dissolved | Completely dissolved | Completely dissolved |
| 0.5 | | 17.1 | 807 | 183 | n.d. |
| 1 | | 19.2 | 980 | 245 | 160 |
| 1.5 | | 23.0 | 1082 | 323 | 239 |
| 2 | | 41.6 | 1101 | 436 | 322 |
| 4 | | 173.6 | 1243 | 931 | 922 |
| 8 | | 776.1 | 1330 | 1386 | 1892 |
| 12 | | 1390.4 | 1255 | 1434 | 2251 |
| 18 | | 3008.0 | 1199 | 1415 | 2906 |
| 24 | | 4444.4 | 1154 | 1335 | 3076 |

From Example 24, the transdermal absorption enhancement aid of the present embodiment was found to be effective even when the preparation comprises no transdermal absorption enhancer (the other transdermal absorption enhancer), i.e., the transdermal absorption enhancement aid of the present embodiment can also be usable as a transdermal absorption enhancer, and in this case, the transdermal penetration amount of MZR at the initial stage was particularly excellent.

II-2 The case using hairless mouse skin (Franz cell test) (1)

(Examples 27 to 32 and Comparative Example 10)

25 mg of ACV and 475 mg of the transdermal absorption enhancer of the present embodiment (any one of choline-linoleic acid, choline-oleic acid, choline-isostearic acid, choline-capric acid, choline-benzoic acid, or guanidine-linoleic acid) or an ionic liquid described in Patent Literature 3 or 4 (diethanolamine (DEA) (DEA melting point: 28°C)-glycolic acid) were placed in a test tube, dispersed for 1 minute with a mixer, and then ultrasonicated for 1 hour. Using each obtained dispersion, the Franz cell test using hairless mouse skin (the conditions were the same as in the Franz cell test described above except that hairless mouse skin (Laboskin, Hoshino Laboratory Animals, Inc.; used after being thawed by dipping in running water for 10 minutes) was used instead of the artificial skin) was conducted. The results are shown in Table 9.

[Table 9]

<Table 9>

| | Transdermal penetration amount of ACV [$\mu$g/cm$^2$] | | | | | | |
|---|---|---|---|---|---|---|---|
| | Example 27 | Example 28 | Example 29 | Example 30 | Example 31 | Example 32 | Comparative Example 10 |
| Time [h] | ACV/ choline-linoleic acid =25/475 | ACV/ choline-oleic acid =25/475 | ACV/choline-isostearic acid =25/475 | ACV/ choline-capric acid =25/475 | ACV/ choline-benzoic acid =25/475 | ACV/guanidi ne-linoleic acid =25/475 | ACV/DEA-glycolic acid =25/475 |
| 0.5 | 0.5 | 0.4 | 30.4 | 0.0 | 0.5 | 6.0 | 0.1 |
| 1 | 1.6 | 2.8 | 343.3 | 0.2 | 0.5 | 1.9 | 0.1 |
| 1.5 | 3.6 | 5.3 | 706.0 | 0.4 | 0.6 | 1.7 | 0.1 |
| 2 | 5.7 | 9.5 | 1006.6 | 1.3 | 0.7 | 2.4 | 0.2 |
| 4 | 16.0 | 35.6 | 2285.3 | 10.0 | 1.1 | 26.6 | 0.3 |
| 8 | 47.7 | 192.5 | 3626.1 | 221.6 | 2.2 | 203.9 | 0.8 |
| 12 | 113.7 | 480.0 | 4241.1 | 852.0 | 4.2 | 483.4 | 1.3 |
| 18 | 295.5 | 998.0 | 4992.9 | 1965.4 | 8.7 | 739.8 | 2.4 |
| 24 | 596.7 | 1468.1 | 5408.9 | 2764.9 | 13.2 | 929.0 | 3.5 |

For the hairless mouse skin, the largest transdermal penetration amount was obtained by use of choline-isostearic acid as the transdermal absorption enhancer, followed by choline-capric acid, -oleic acid, and -linoleic acid. ACV also transdermally penetrated when Guanidine-linoleic acid was used.

However, when choline-benzoic acid was used as the transdermal absorption enhancer (Example 31), ACV did not penetrate through the hairless mouse skin much. The hairless mouse skin is more lipophilic than the artificial skin. Therefore, the transdermal penetration amount became higher when the acidic functional group-containing compound b) having higher LogP and higher lipophilicity was used.

On the other hand, ACV could not transdermally penetrate when DEA-glycolic acid described in Patent Literature 3 or 4 was used as the transdermal absorption enhancer.

(Examples 33, 39, and 40)

25 mg of MZR, 25 mg of the transdermal absorption enhancement aid of the present embodiment (choline-linoleic acid), 3000 mg, 450 mg, or 225 mg of the transdermal absorption enhancer (the other transdermal absorption enhancer) (IPM or linoleic acid), and 225 mg of white petrolatum (only in Example 40) were placed in a test tube as compositions shown in Table 10, dispersed for 1 minute with a mixer, and then ultrasonicated for 1 hour.

Using each obtained dispersion, the Franz cell test using hairless mouse skin (the conditions were the same as in Examples 27 to 32 except that Laboskin was used as the hairless mouse skin after being thawed at room temperature for 60 minutes) was conducted. The results are shown in Table 10.

[Table 10]

<Table 10>

| Time [h] | Transdermal penetration amount of MZR[μg/cm²] | | |
| | Example 33 | Example 39 | Example 40 |
| | M Z R / choline-linoleic acid / IPM =25/25/3000 | M Z R / choline-linoleic acid / linoleic acid =25/25/450 | M Z R / choline-linoleic acid / IPM / white petrolatum =25/25/225/225 |
|---|---|---|---|
| 0 | 0.0 | 0.0 | 0.0 |
| 0.5 | 0.2 | 0.0 | 7.7 |
| 1 | 0.5 | 0.1 | 6.7 |
| 1.5 | 1.7 | 0.2 | 13.8 |
| 2 | 5.1 | 0.4 | 22.1 |
| 4 | 68.6 | 3.0 | 76.5 |
| 8 | 419.3 | 13.1 | 155.0 |
| 12 | 1616.7 | 25.5 | 212.8 |
| 18 | 2622.6 | 47.6 | 277.7 |
| 24 | 3186.1 | 70.8 | 292.7 |

(Examples 41 to 51)

25 mg of MZR and 475 mg of the transdermal absorption enhancer of the present embodiment (any one of choline-linoleic acid, choline-oleic acid, choline-isostearic acid, choline-capric acid, choline-propionic acid, choline-DL-lactic acid, choline-glutamic acid, histamine-oleic acid, histamine-linoleic acid, guanidine-oleic acid, or guanidine-linoleic acid) were placed in a test tube, dispersed for 1 minute with a mixer, and then ultrasonicated for 1 hour. Using each obtained dispersion, the Franz cell test using hairless mouse (the conditions were the same as in Examples 27 to 32) was conducted. The results are shown in Table 11.

In Example 41, the transdermal penetration amount of MZR was increased as compared with Example 33 using the equimolar salt (choline-linoleic acid) as the transdermal absorption enhancement aid. This is probably because the content of the equimolar salt (choline-linoleic acid) in the dispersion was high. The transdermal penetration amount of MZR when choline-oleic acid was used is close to that when choline-linoleic acid was used, and the transdermal penetration amount of MZR at the initial stage when choline-isostearic acid was used is higher than that when choline-linoleic acid was used. MZR transdermally penetrated when choline-capric acid was used.

However, in Examples 45 to 47 using the acidic functional group-containing compound b) having lower LogP than that of fatty acid (propionic acid (LogP: 0.3), DL-lactic acid (LogP: -0.7), glutamic acid (LogP: -3.7)), MZR did not transdermally penetrate much. MZR was dissolved in the equimolar salts used in these Examples. Therefore, in these Examples, the equimolar salt had poor affinity for the hairless mouse skin due to the low LogP of the acidic functional group-containing compound b) constituting the equimolar salt, and might hinder the transdermal penetration.

In Examples 48 to 51 using histamine or guanidine as the basic functional group-containing compound a) instead of choline, MZR transdermally penetrated as in Examples 41 and 42. The largest transdermal penetration amount of MZR was obtained by use of histamine as the basic functional group-containing compound a), followed by guanidine-linoleic acid and choline-linoleic acid. The reason why a large transdermal penetration amount of MZR was obtained when histamine was used is considered to be due to the influence of the vascular penetrability-increasing effect of histamine.

(Example 52)

Transdermal penetrability was examined (Example 52) in the same way as in Example 41 except that an equimolar salt (imidazole-isostearic acid), wherein imidazole (molecular weight: 68, melting point: 90°C) was used as the basic functional group-containing compound a) and isostearic acid was used as the acidic functional group-containing compound b), was used as the transdermal absorption enhancer. As a result, it was found that MZR could transdermally penetrate in this case, albeit poorer than the level of transdermal penetration when choline-isostearic acid was used.

[Table 11]

<Table 11>

| Time [h] | Example 41 | Example 42 | Example 43 | Example 44 | Example 45 | Example 46 | Example 47 | Example 48 | Example 49 | Example 50 | Example 51 | Example 52 |
|---|---|---|---|---|---|---|---|---|---|---|---|---|
| | **MZR/choline-li-noleic acid =25/475** | **MZR/choline-oleic acid =25/475** | **MZR/choline-isostearic acid =25/475** | **MZR/choline-ca-pric acid =25/475 ca-pric acid** | **MZR/choline -propionic acid =25/475** | **MZR/choline-DL-lactic acid =25/475** | **MZR/choline -glutamic acid =25/475** | **MZR/histamine -oleic acid =25/475** | **MZR/hista-mine -lino-leic acid =25/475** | **MZR/ guanidine -oleic acid =25/475** | **MZR/guanidine-linoleic acid =25/475** | **MZR/ imida-zole -iso-stearic acid =25/475** |
| | MZR was dissolved | MZR was dissolved | MZR was dissolved | MZR was dissolved | MZR was dissolved | MZR was dissolved | MZR was partially dis-solved | MZR was dis-solved | MZR was dis-solved | MZR was dis-solved | MZR was dis-solved | MZR was par-tially dis-solved |
| 0.5 | 9.6 | 6.3 | 140.6 | 13.0 | 1.6 | 0.0 | 0.0 | 20.6 | 13.4 | 4.6 | 5.2 | 3.0 |
| 1 | 5.2 | 2.6 | 762.4 | 10.4 | 0.6 | 0.0 | 0.0 | 58.1 | 72.2 | 17.3 | 3.7 | 3.0 |
| 1.5 | 11.0 | 4.8 | 1434.4 | 17.5 | 0.7 | 0.0 | 0.0 | 126.1 | 220.9 | 69.3 | 6.4 | 7.2 |
| 2 | 27.0 | 12.5 | 1888.8 | 28.8 | 1.3 | 0.0 | 0.0 | 243.0 | 478.5 | 164.8 | 13.2 | 15.3 |
| 4 | 232.0 | 171.9 | 3593.3 | 289.6 | 1.1 | 0.0 | 0.0 | 1355.6 | 2474.0 | 763.4 | 196.7 | 141.2 |
| 8 | 1297.3 | 1315.1 | 4775.8 | 2794.0 | 1.4 | 0.0 | 0.0 | 4276.1 | 4823.6 | 1332.4 | 2071.7 | 563.2 |
| 12 | 2754.3 | 2420.1 | 4902.3 | 3556.8 | 2.2 | 0.1 | 0.2 | 6058.1 | 5849.7 | 1531.0 | 3891.2 | 897.8 |
| 18 | 4804.0 | 3939.4 | 5022.9 | 3642.0 | 5.3 | 0.1 | 0.4 | 7381.3 | 7480.1 | 1805.4 | 5902.9 | 1520.1 |
| 24 | 5686.3 | 4752.9 | 4808.8 | 3214.1 | 12.8 | 0.3 | 0.5 | 7040.1 | 7976.3 | 1960.8 | 6876.0 | 3124.0 |

(Comparative Examples 12 to 22)

25 mg of MZR and 475 mg of each conventional transdermal absorption enhancer (azone, limonene, octyldodecanol, ethanol, isopropyl alcohol (IPA), alkali water (pH 12.36), or DMSO) or ionic liquids described in Patent Literature 3 or 4 (TIPA (triisopropanolamine, molecular weight: 191)-capric acid, DIPA (diisopropanolamine, molecular weight: 133, melting point: 42-45°C)-isostearic acid, DEA (melting point: 28°)-isostearic acid, or $\alpha$-GPC ($\alpha$-glycerylphosphoryl-choline, molecular weight: 258, melting point: 61°C)-linoleic acid) were placed in a test tube, dispersed for 1 minute with a mixer, and then ultrasonicated for 1 hour. Using obtained dispersion, the Franz cell test using hairless mouse skin (the conditions were the same as in Examples 27 to 32) was conducted. The results are shown in Table 12.

Though MZR could also transdermally penetrate when azone, limonene, IPA, and DEA-isostearic acid were used as the transdermal absorption enhancer, the effect was inferior to that of the transdermal absorption enhancer of the present embodiment.

[Table 12]

<Table 12>

| Time [h] | Transdermal penetration amount of MZK μg/cm² | | | | | | | | | | |
|---|---|---|---|---|---|---|---|---|---|---|---|
| | Comparative Example 12 | Comparative Example 13 | Comparative Example 14 | Comparative Example 15 | Comparative Example 16 | Comparative Example 17 | Comparative Example 18 | Comparative Example 19 | Comparative Example 20 | Comparative Example 21 | Comparative Example 22 |
| | MZR/ Azone=25/475 | MZR/ limonene =25/475 | MZR/ octyldo-decanol =25/475 | MZR/ etha-nol =25/475 | MZR/IPA=25/475 | MZR/alkali water =25/475 | MZR/DMSO=25/ 475 | MZR/TIPA-capric acid =25/475 | MZR/DIPA-isostearic acid =25/475 | MZR/DEA-isostearic acid =25/475 | MZR/α GPC-linoleic acid =25/475 |
| | MZR was partially dissolved | MZR was partially dis-solved | MZR was insolu-ble | MZR was in-soluble | MZR was partially dissolved | MZR was dis-solved | MZR was dis-solved | MZR was in-soluble | MZR was in-soluble | MZR was partially dis-solved | MZR was in-soluble |
| 0.5 | 6.2 | 32.3 | 1.7 | 7.4 | 3.2 | 1.1 | 7.0 | 2.9 | 7.5 | 32.9 | 0.0 |
| 1 | 8.0 | 10.3 | 4.3 | 2.3 | 1.5 | 0.5 | 7.3 | 2.0 | 2.9 | 14.1 | 0.0 |
| 1.5 | 18.6 | 12.8 | 7.0 | 2.0 | 1.6 | 0.6 | 9.1 | 2.3 | 2.0 | 11.9 | 0.0 |
| 2 | 38.4 | 18.6 | 9.3 | 2.5 | 1.5 | 0.7 | 8.9 | 3.0 | 2.5 | 15.3 | 0.0 |
| 4 | 245.0 | 64.6 | 24.3 | 20.9 | 18.3 | 1.5 | 17.6 | 7.7 | 8.1 | 40.2 | 0.0 |
| 8 | 1074.9 | 266.8 | 65.0 | 136.9 | 296.8 | 3.5 | 24.7 | 20.5 | 72.0 | 152.4 | 0.6 |
| 12 | 2765.1 | 777.1 | 99.6 | 252.1 | 488.1 | 5.6 | 34.1 | 32.7 | 163.1 | 319.8 | 1.9 |
| 18 | 4142.6 | 1498.4 | 158.9 | 338.9 | 701.0 | 10.3 | 47.2 | 59.6 | 292.6 | 572.4 | 6.0 |
| 24 | 4397.7 | 1885.5 | 208.8 | 441.2 | 840.7 | 16.3 | 55.2 | 104.5 | 410.8 | 821.0 | 16.8 |

(Examples 53 to 58 and Comparative Examples 23 to 26)

25 mg of methotrexate (MTX, LogP: -1.8, molecular weight: 454) and 475 mg of the transdermal absorption enhancer of the present embodiment (any one of choline-oleic acid, choline-isostearic acid, choline-linoleic acid, choline-capric acid, or guanidine-linoleic acid), ionic liquids described in Patent Literature 3 or 4 (TIPA-isostearic acid or DEA-glycolic acid), or azone were placed in a test tube, dispersed for 1 minute with a mixer, and then ultrasonicated for 1 hour. Using each obtained dispersion, the Franz cell test using hairless mouse skin (the conditions were the same as in Examples 27 to 32) was conducted. The results are shown in Table 13.

In Examples using choline-oleic acid, choline-isostearic acid, choline-linoleic acid, choline-capric acid, or guanidine-linoleic acid as the transdermal absorption enhancer, MTX could transdermally penetrate. However, in Example 58 (choline-benzoic acid) using benzoic acid as the acidic functional group-containing compound b) having lower LogP than that of fatty acid, MTX did not transdermally penetrate much. This equimolar salt had poor affinity for the hairless mouse skin due to the low LogP of the acidic functional group-containing compound b), and might hinder the transdermal penetration.

On the other hand, Comparative Example 23 (TIPA-isostearic acid) was inferior in the transdermal penetration amount of MTX to its counterpart Example 54. In Comparative Example 24 (DEA-glycolic acid) and Comparative Example 25 (azone), MTX hardly transdermally penetrated.

[Table 13]

<Table 13>

| | Transdermal penetration amount of MTX [$\mu$g/cm$^2$] | | | | | | | | |
|---|---|---|---|---|---|---|---|---|---|
| | Example 53 | Example 54 | Example 55 | Example 56 | Example 57 | Example 58 | Comparative Example 23 | Comparative Example 24 | Comparative Example 25 |
| | **MTX/ choline-oleic acid =25/475** | **choline-MTX/ isostearic acid =25/475** | **MTX/choline-linoleic acid =25/475** | **MTX/choline-capric acid =25/475** | **MTX/guanidine linoleic acid = 25/475** | **MTX/ choline benzoic acid =25/475** | **MTX/TIPA isostearic acid =25/475** | **MTX/DEA-glycolic acid =25/475** | **MTX/Azone= 25/475** |
| | MTX was dissolved | MTX was partially dissolved | MTX was dissolved | MTX was dissolved | MTX was partially dissolved | MTX was insoluble | MTX was insoluble | MTX was dissolved | MTX was insoluble |
| **0** | **0** | **0** | **0** | **0** | **0** | **0** | **0** | **0** | **0** |
| **0.5** | **0.06** | **0.99** | **0.15** | **0.04** | **0.02** | **0.22** | **18.29** | **34.68** | **0.16** |
| **1** | **0.22** | **5.04** | **0.93** | **0.11** | **0.05** | **0.37** | **7.39** | **10.33** | **0.11** |
| **1.5** | **0.76** | **8.71** | **2.32** | **0.52** | **0.11** | **0.49** | **6.43** | **8.62** | **0.23** |
| **2** | **1.96** | **13.40** | **4.08** | **1.27** | **0.31** | **0.60** | **6.18** | **8.33** | **0.36** |
| **4** | **26.10** | **178.23** | **29.29** | **11.24** | **12.06** | **1.27** | **8.27** | **8.36** | **4.38** |
| **8** | **341.57** | **1095.70** | **245.29** | **534.41** | **232.47** | **1.85** | **31.59** | **8.39** | **16.58** |
| **12** | **827.03** | **1666.91** | **673.77** | **1230.16** | **536.00** | **2.46** | **141.34** | **8.51** | **29.81** |
| **18** | **1439.98** | **2226.81** | **1501.59** | **1579.35** | **898.71** | **6.57** | **368.86** | **8.74** | **50.73** |
| **24** | **1789.73** | **2468.41** | **2553.66** | **1368.03** | **1009.59** | **10.21** | **527.31** | **8.90** | **70.21** |

II-3 The case using hairless rat skin (PK test)

The MZR transdermal preparations of Examples 59 and 60 formulated using MZR and the transdermal absorption enhancer of the present embodiment and Comparative Example 26 as given below were evaluated for their transdermal absorbability when administered to rats. Hairless rats (Japan SLC, Inc.) were used for the evaluation of the transdermal absorbability.

- Hairless rats (HWY/SIc) 8 weeks old at the time of purchase and 10 weeks old at the time of the test

These rats are Wistar rats having the trait of hair abnormality due to the autosomal semidominant gene (HI) and are generally used for evaluating the transdermal absorbability of transdermal absorption-type pharmaceutical products.

- Measurement method: MZR concentration in plasma was measured by LC-MS/MS.
- Usage

Each MZR test preparation was transdermally administered by a single administration.

- Dose

The dose was set to 15 mg/kg because the no-observed-adverse-effect level (NOAEL) of MZR in subcutaneous administration to rats is 5 mg/kg and the rate of penetration is approximately 30% in an *in vitro* penetration test using hairless mouse skin.

(Example 59)

30 mg of MZR and 562 mg of the transdermal absorption enhancer of the present embodiment (choline-linoleic acid) were respectively weighed, then added to a glass vial, and mixed using a microspatula to the extent that an aggregated mass of MZR was not observed. Then, the mixture was ultrasonicated for 1 hour in a hot bath adjusted to 40°C, and then left to stand overnight at room temperature to prepare an administration base for 8 rats. This preparation was carried out on the day before administration. A lint (Pip-Fujimoto Co., Ltd.) cut into a circle having a diameter of 1.5 cm was laminated onto Elastopore No. 50 (Nichiban Co., Ltd.) cut into a circle having a diameter of 3 cm to prepare a material for pasting. The administration base was applied onto the material for pasting to prepare a test preparation.

The test preparation was pasted onto the central portion of the back of each of 3 hairless rats under isoflurane anesthesia and then fixed with a bandage and a press-net No.2. An Elizabethan collar was put on each rat so as to prevent the rat from detaching the base during the experiment.

1 hour, 4 hours, 8 hours, and 24 hours after the transdermal administration, heparin blood collection was performed through the subclavian vein under isoflurane anesthesia to collect approximately 100 μL of blood. The blood was centrifuged (4°C, 10000 rpm). Then, the plasma components were collected and stored in a freezer of -30°C until subjected to analysis. To 20 μL of the plasma measurement sample, a calibration curve sample, or a QC sample, 20 μL of ultrapure water (Milli-Q water), 40 μL of an internal standard solution (internal standard: 2'-deoxyuridine; a 10 mM DMSO solution was prepared and then serially diluted with Milli-Q water to prepare 5 μM aqueous solutions), and 320 μL of a 0.1% acetic acid solution were added, and the mixture was stirred. The whole amount of this sample was added to an ultrafiltration filter and centrifuged (approximately 6800 G, room temperature, 15 min), and 2 μL of the filtrate was injected to LC-MS/MS.

The formulation of the administration base is shown in Table 14, and the MZR concentrations in the plasmas obtained 1 hour, 4 hours, 8 hours, and 24 hours after the transdermal administration are shown in Figure 3(b).

LC-MS/MS analysis conditions

(HPLC conditions)

Column: Inertsil ODS-3, 5 μm, 2.1 × 150 mm (GL Sciences Inc.)

Guard column: Inertsil ODS-3, 5 μm, 3.0 id × 10 mmL (GL Sciences Inc.)

Mobile phase: 0.1% acetic acid/acetonitrile = 95/5

Flow rate: 0.2 mL/min

Injection volume: 2 μL (MS/MS conditions)

Ion source: Turbo ion Spray

Polarity: Negative

Scan type: Multi Reaction Monitoring (MRM)

MS Instrument setup:

[Table 14]

| Curtain Gass | Source Gas Temp(°C) | Nevulizer Gas (GS1) | Turbo Gas (GS2) | Ionspray Voltage | Collision Gas |
|---|---|---|---|---|---|
| 10 | 250 | 30 | 20 | -4200 | 6 |

Monitor ions and conditions:

[Table 15]

| Compound | Q1 MS (Da) | Q3 MS (Da) | Declustering Potential (DP) | Collision Energy (CE) | Collision Cell Exit Potential (CXP) |
|---|---|---|---|---|---|
| Mizoribine | 257.9 | 125.9 | -65 | -24 | -9 |
| IS | 227.1 | 183.9 | -75 | -16 | -11 |

(Example 60)

An administration base was prepared in the same way as in Example 59 except that the amount of choline-linoleic acid added as the transdermal absorption enhancer/enhancement aid was changed to 296 mg and 89 mg of white petrolatum and 178 mg of octyldodecanol were further added as the other transdermal absorption enhancers. Transdermal absorbability was also evaluated. The formulation of the administration base is shown in Table 14, and the MZR concentrations in the plasmas obtained 1 hour, 4 hours, 8 hours, and 24 hours after the transdermal administration are shown in Figure 3(c).

(Comparative Example 26)

An administration base was prepared in the same way as in Example 59 except that choline-linoleic acid as the transdermal absorption enhancer of the present embodiment was not added and 385 mg of white petrolatum and 178 mg of octyldodecanol were added as the other transdermal absorption enhancers. Transdermal absorbability was also evaluated. The formulation of the administration base is shown in Table 14, and the MZR concentrations in the plasmas obtained 1 hour, 4 hours, 8 hours, and 24 hours after the transdermal administration are shown in Figure 3(a).

[Table 16]

<Table 14>

| Formulation of administration base | Example 59 | Example 60 | Comparative Example 26 |
|---|---|---|---|
| Mizoribine (MZR) [mg] | 3.7 | 3.7 | 3.7 |
| Choline-linoleic acid [mg] | 70.3 | 37 | |
| Octyldodecanol [mg] | | 22.2 | 22.2 |
| White petrolatum [mg] | | 11.1 | 48.1 |

(continued)

| Formulation of administration base | Example 59 | Example 60 | Comparative Example 26 |
|---|---|---|---|
| Preparation mass [mg] | 74 | 74 | 74 |
| Drug content [mass%] | 5 | 5 | 5 |

For the formulation, to which the transdermal absorption enhancer of the present embodiment was not added, (Comparative Example 26), no concentration of MZR in the plasmas was observed (Figure 3(a)). By contrast, for the formulations using the transdermal absorption enhancer or enhancement aid of the present embodiment (Examples 59 and 60), the transdermal absorbability-improving effect on MZR was observed (Figures 3(b) and (c)), demonstrating the effectiveness of the transdermal absorption enhancer of the present embodiment and enhancement aid.

II-4 The case using hairless mouse skin (Franz cell test) (2)

(Examples 61 and 62 and Comparative Example 27)

25 mg of cimetidine (LogP = 0.4) and 475 mg of the transdermal absorption enhancer of the present embodiment (either of choline-isostearic acid or choline-capric acid) or TIPA-isostearic acid described in Patent Literature 4 were placed in a test tube, dispersed for 1 minute with a mixer, and then ultrasonicated for 1 hour. Using each obtained dispersion, the Franz cell test (the conditions were the same as in Examples 27 to 32) was conducted. The results are shown in Table 15. The dermal penetration of cimetidine was observed in Examples 61 and 62, whereas the transdermal penetration of cimetidine was not observed in Comparative Example 27.

[Table 17]

<Table 15>

| | Transdermal penetration amount of cimetidine [$\mu g/cm^2$] | | |
|---|---|---|---|
| | Example 61 | Example 62 | Comparative Example 27 |
| | **Cimetidine/choline-isostearic acid =25/475** | **Cimetidine/choline-capric acid =25/475** | **Cimetidine/TIPA-isostearic acid =25/475** |
| | Cimetidine was dissolved | Cimetidine was dissolved | Cimetidine was insoluble |
| **0.5** | **28.5** | **0.0** | **0.0** |
| **1** | **87.6** | **0.0** | **0.0** |
| **1.5** | **147.2** | **0.0** | **0.0** |
| **2** | **227.7** | **0.0** | **0.0** |
| **4** | **793.6** | **19.7** | **0.2** |
| **8** | **1698.7** | **171.5** | **0.7** |
| **12** | **2284.9** | **525.7** | **1.2** |
| **18** | **2836.0** | **1165.7** | **2.1** |
| **24** | **3151.4** | **1491.9** | **2.9** |

(Example 63)

25 mg of carbidopa (LogP = -2.2), 425.5 mg of choline-isostearic acid as the transdermal absorption enhancer of the present embodiment, and 49.5 mg of lactic acid were mixed in advance to adjust the pH to 8.08, and this mixture was placed in a test tube, dispersed for 1 minute with a mixer, and then ultrasonicated for 1 hour. Using each obtained dispersion, the Franz cell test using hairless mouse skin (the conditions were the same as in Examples 27 to 32) was conducted.

The results are shown in Table 16. The transdermal penetration of carbidopa was observed when choline-isostearic acid was used as the transdermal absorption enhancer of the present embodiment.

If a transdermal preparation has pH as high as 11 by using the transdermal absorption enhancer of the present embodiment (e.g., choline-isostearic acid) alone, the pH is adjusted to around from 8 to 9 by the addition of an acid such as lactic acid in an amount corresponding to 1/3 to 1/2 of the mole of the transdermal absorption enhancer of the present embodiment. As a result, reduction in dermal irritation and the suppression of drug decomposition caused by high pH can be achieved.

[Table 18]

Table 18

<Table 16>

| | Transdermal penetration amount of carbidopa [$\mu$g/cm$^2$] |
|---|---|
| | Example 63 |
| | **Carbidopa/choline-isostearic acid/lactic acid =25/425.5/49.5** |
| | Carbidopa was dissolved |
| 0.5 | 0.2 |
| 1 | 0.3 |
| 1.5 | 0.6 |
| 2 | 1.0 |
| 4 | 8.1 |
| 8 | 64.1 |
| 12 | 178.9 |
| 18 | 1087.1 |
| 24 | 2548.1 |

(Example 64)

25 mg of lisinopril (LogP = -3.0) and 475 mg of choline-isostearic acid as the transdermal absorption enhancer of the present embodiment were placed in a test tube, dispersed for 1 minute with a mixer, and then ultrasonicated for 1 hour. Using the obtained dispersion, the Franz cell test using hairless mouse skin (the conditions for the hairless mouse skin were the same as in Examples 27 to 32) was conducted.

The results are shown in Table 17. The transdermal penetration of lisinopril was observed by use of choline-isostearic acid as the transdermal absorption enhancer of the present embodiment.

[Table 19]

<Table 17>

| | Transdermal penetration amount of lisinopril [$\mu$g/cm$^2$] |
|---|---|
| | Example 64 |
| | Lisinopril/choline-isostearic acid = 25/475 |
| | Lisinopril was almost dissolved |
| 0.5 | 164.8 |
| 1 | 444.7 |

(continued)

| | Transdermal penetration amount of lisinopril [$\mu$g/cm$^2$] |
|---|---|
| | Example 64 |
| 1.5 | 828.3 |
| 2 | 1210.9 |
| 4 | 2669.0 |
| 8 | 4087.6 |
| 12 | 4441.0 |
| 18 | 4837.8 |
| 24 | 4896.9 |

III Relationship between molecular weight and melting point of basic functional group-containing compound a) and solubility and dermal penetrability of water-soluble active ingredient

On the basis of the Examples and Comparative Examples described above, as to each equimolar salt constituted by the basic functional group-containing compound a) and the acidic functional group-containing compound b) (isostearic acid or linoleic acid), the molecular weight and the melting point of the basic functional group-containing compound a), the solubility of MZR in the equimolar salt, and the dermal penetrability (hairless mouse skin) of MZR in the case of using the equimolar salt as the transdermal absorption enhancer were summarized in Table 18.

As shown in Table 18, when the basic functional group-containing compound a) satisfied both conditions of a molecular weight of from 50 to 120 and a melting point of from 50 to 350°C, the equimolar salt dissolved MZR and the MZR transdermally penetrated. This is probably because the molecular weight is related to affinity for water-soluble active ingredients and the melting point is related to affinity for the skin.

[Table 20]

<Table 18>

| | Basic functional group-containing compound | Presence or absence *in vivo* | Molecular weight <dominant factor for affinity for water-soluble API> | Melting point (°C) <dominant factor for affinity for skin> | MZR dissolution | Transdermal penetrability of MZR |
|---|---|---|---|---|---|---|
| | | | 50-120 | 50-310 | | |
| Non Patent Literature 1 | [C1 mim][(MeO)$_2$PO$_2$]1,3-Dimethylimidazolium Dimethyl Phosphate | × | 132.59 | 125 (Cl salt) | Insoluble | Not penetrated by IL alone |
| Patent Literature 2 | 1-Ethyl-3-methylimidazolium trifluoromethanesulfonate | × | 146.62 | 78 (Cl salt) | Insoluble | Not penetrated |
| Patent Literature 4 | TIPA | × | 191.27 | 50.00 | Insoluble | Not penetrated |
| Patent Literature 4 | DIPA | × | 133.2 | 45 | Insoluble | Not penetrated |
| Patent Literature 4 | DEA | × | 105.14 | 28 | Insoluble | Not penetrated |
| Example of present application | Choline | ○ | 104.17 | 305 | Dissolved | Penetrated |
| Example of present application | Histamine | ○ | 111.15 | 83.5 | Dissolved | Penetrated |
| Example of present application | Guanidine | ○ | 59.07 | 50 | Dissolved | Penetrated |
| Comparative Example of present application | αGPC (glycerylphosphorylcholine) | × | 258.23 | 61 | Insoluble | Not penetrated |
| Reference Example | Adenine | ○ | 135.13 | 360 | Insoluble | Not penetrated |
| Reference Example | 1-Imidazole acetate | ○ | 126.11 | 258.5 | Insoluble | Not penetrated |

EP 3 395 369 A1

34

IV Relationship between LogP of basic functional group-containing compound b) and solubility and dermal penetrability of water-soluble active ingredient

On the basis of Examples and Comparative Examples described above, as to each equimolar salt constituted by the basic functional group-containing compound a) (choline) and the acidic functional group-containing compound b), the LogP of the acidic functional group-containing compound b), the solubility of MZR in the equimolar salt, and the dermal penetrability (artificial skin and hairless mouse skin) of MZR in the case of using the equimolar salt as the transdermal absorption enhancer, etc., were summarized in Table 19.

As shown in Table 19, it was confirmed that there is a relationship between the LogP of the acidic functional group-containing compound b) and the transdermal penetration of the active ingredient. In short, for the artificial skin, the transdermal penetration was observed when the LogP was from -4 to 2.0 for the artificial skin, and for the hairless mouse skin which is more hydrophobic than the artificial skin, the transdermal penetration was observed when the LogP was from 2.0 to 7.3. Thus, the optimum LogP of the acidic functional group-containing compound b) differs depending on the hydration state of the skin. Therefore, the LogP is preferably selected as an appropriate value within the range of from -4 to 7.3.

[Table 21]

Table 21

<Table 19>

| Acidic functional group-containing compound | State | Appearance ×: Solid △: Gel ○: Liquid | LogP <value of XLogP3⟩ | pKa | M.W. | m·P (°C) | Water solubility | Transdermal penetrability of MZR (artificial skin) | Transdermal penetrability of MZR (hairless mouse) |
|---|---|---|---|---|---|---|---|---|---|
| Stearic acid | Solid | × | 7.40 | 4.75 | 284.48 | 69.6 | Practically insoluble in water (0.0003%, 20°C) | Not penetrated | Not penetrated |
| Isostearic acid | Liquid | ○ | 7.20 | 4.90 | 284.48 | 16 | 0.1-1 g/100 mL at 23°C | Not penetrated | Penetrated |
| Linoleic acid | Liquid | △ | 6.80 | 4.77 | 280.45 | -5 | Insoluble | Not penetrated | Penetrated |
| Oleic acid | Liquid | △ | 6.50 | 5.02 | 282.46 | 16.3 | negligible | Not penetrated | Penetrated |
| Palmitic acid | Solid | × | 6.40 | 4.75 | 256.42 | 63 | Insoluble | Not penetrated | Penetrated |
| Myristic acid | Solid | × | 5.30 | 4.90 | 228.37 | 54.4 | <0.1 g/100 mL at 18°C | Not penetrated | Penetrated |
| Dodecanoic acid | Solid | × | 4.20 | 5.30 | 200.32 | 45 | 5.5 mg/100 g (20°C) | Not penetrated | Penetrated |
| Capric acid | Solid | × - △ | 4.10 | 4.90 | 172.26 | 31 | 0.015 g/100 g (20°C) | Not penetrated | Penetrated |
| Benzoic acid | Solid | ○ | 1.90 | 4.20 | 122.12 | 122.35 | 0.34 g/100 ml (25°C) | Penetrated | Not penetrated |
| Isobutyric acid | Liquid | ○ | 0.80 | 4.84 | 88.11 | -47 | 210 g/L (20°C) | Penetrated | Not penetrated |
| Propionic acid | Liquid | ○ | 0.30 | 4.88 | 74.08 | -21 | 37 g/100 mL | Penetrated | Not penetrated |
| Adipic acid | Solid | × | 0.10 | 4.43, 5.41 | 146.14 | 152 | 1.4 g/100 mL | Penetrated | Not penetrated |
| Acetic acid | Liquid | × | -0.20 | 4.76 | 60.05 | 16.7 | miscible (1000000 mg/L (at 25°C)) | Penetrated | Not penetrated |
| Vanillylmandelic acid | Solid | × - △ | -0.20 | 3.11 | 198.17 | 89 | H2O: ~50 mg/mL | Penetrated | Not penetrated |
| Maleic acid | Solid | ○ | -0.30 | 1.92, 6.58 | 116.07 | 131 | 78 g/100 mL (25°C) | Penetrated | Not penetrated |

(continued)

| Acidic functional group-containing compound | State | Appearance ×: Solid △: Gel ○: Liquid | LogP <value of XLogP3⟩ | pKa | M.W. | m·P (°C) | Water solubility | Transdermal penetrability of MZR (artificial skin) | Transdermal penetrability of MZR (hairless mouse) |
|---|---|---|---|---|---|---|---|---|---|
| Glutaric acid | Solid | ○ | -0.30 | 4.34, 5.42 | 132.11 | 96.5 | 430 g/L (20°C) | Penetrated | Not penetrated |
| Fumaric acid | Solid | × | -0.30 | 3.03 | 116.07 | 200 | 0.63 g/100 mL-water (25°C) | Penetrated | Not penetrated |
| Succinic acid | Solid | ○ | -0.60 | 4.19, 5.48 | 118.09 | 186 | 58 g/L (20°C) | Penetrated | Not penetrated |
| DL-Lactic acid | Liquid | ○ | -0.70 | 3.86 | 99.08 | 16.8 | 876 g/L | Penetrated | Not penetrated |
| Glycolic acid | Solid | ○ | -1.10 | 3.83 | 76.05 | 75 | 10 g/100 mL | Penetrated | Not penetrated |
| Glutamic acid | Solid | ○ | -3.7 | 2.2, 4.3, 9.7 | 147.13 | 199 | 1.72 g/100 g (20°C) | Penetrated | Not penetrated |

V Tg or melting point of the equimolar salt

The Tgs or melting points of the equimolar salts used in Examples and Comparative Examples described above are shown in Table 20.

For the Tg and melting point measurement, DSC (TA Instruments Q2000 DSC) was used. 5 mg of a sample was enclosed in an aluminum pan, cooled at a rate of -10°C/min from room temperature to -90°C, and then heated to 90°C at a rate of 10°C/min, and the temperature of the endothermic peak was read. The temperature, around which the baseline shifts, was determined to be the Tg, while the temperature, around which the baseline does not shift, was determined to be the melting point.

[Table 22]

<Table 20>

| | Equimolar salt | Tg or melting point (°C) |
|---|---|---|
| Example | Choline-isostearic acid | -61.7 |
| | Choline-linoleic acid | -14.5 |
| | Choline-oleic acid | -31 |
| | Choline-capric acid | 48.6 |
| | Choline-benzoic acid | - |
| | Histamine-linoleic acid | -40 |
| | Histamine-oleic acid | -30 |
| | Guanidine-linoleic acid | -59.8 |
| | Guanidine-oleic acid | -40.8 |
| Comparative Example | TIPA-isostearic acid | -56.6 |
| | TIPA-capric acid | 37.5 |
| | DIPA-isostearic acid | -60 |
| | DEA-isostearic acid | -60.9 |
| | DEA-glycolic acid | -60.8 |

VI NMR Measurement Example

The nuclear magnetic resonance spectrum measurement of the equimolar salts used in Examples and Comparative Examples described above was carried out.

The nuclear magnetic resonance (NMR) spectra were measured using AVANCE II (manufactured by Bruker Corp.) under the following conditions:

Resonance frequency: $^1$H 400 MHz, $^{13}$C 100 MHz

Solvent: deuterated methanol ($CD_3OD$)

Measurement temperature: room temperature (25°C)

Chemical shift values were determined based on the deuterated methanol-derived signals 1H-NMR 3.31 ppm (quint) and 13C-NMR 49.0 ppm (sept).

The results are shown below.

[Table 23]

| | | 1H-NMR |
|---|---|---|
| | | Chemical shift value δ [ppm] |
| 1 | Choline-isostearic acid | 4.03-3.99 (m, 2H), 3.52-3.49 (m, 2H), 3.23 (s, 9H), 2.15 (td, J=9.0, 3.5Hz, 1H), 1.53 (dt, J=9.0, 7.2Hz, 2H), 1.32-1.29 (28H), 0.90 (t, J=7.0Hz, 3H), 0.89 (t, J=7.0Hz, 3H) |
| 2 | Choline-linoleic acid | 5.40-5.28 (4H), 4.02-3.98 (m, 2H), 3.51-3.49 (m, 2H), 3.22 (s, 9H), 2.78 (t, J=6.4Hz, 2H), 2.15 (t, J=7.6Hz, 2H), 2.06 (dt, J=6.8, 6.4Hz, 4H), 1.60 (quint, J=6.8Hz, 2H), 1.34-1.29 (14H), 0.91 (t, J=7.0Hz, 3H) |
| 3 | Choline-oleic acid | 5.38-5.30 (2H), 4.02-3.98 (m, 2H), 3.51-3.49 (m, 2H), 3.22 (s, 9H), 2.15 (t, J=7.6Hz, 2H), 2.09-1.97 (m, 4H), 1.60 (quint, J=7.2Hz, 2H), 1.33-1.28 (20H), 0.90 (t, J=6.8Hz, 3H) |
| 4 | Choline-capric acid | 4.02-3.98 (m, 2H), 3.51-3.49 (m, 2H), 3.22 (s, 9H), 2.15 (t, J=7.8Hz, 2H), 1.59 (quint, J=7.3Hz, 2H), 1.32-1.30 (12H), 0.90 (t, J=7.0Hz, 3H) |
| 5 | Choline-benzoic acid | 7.96 (dd, J=6.8, 1.2Hz, 2H), 7.43-7.34 (m, 3H), 3.98-3.94 (m, 2H), 3.45-3.42 (m, 2H), 3.15 (s, 9H) |
| 6 | Histamine-linoleic acid | 7.65 (d, J=1.0Hz, 1H), 6.97 (d, J=1.0Hz, 1H), 5.39-5.28 (4H), 3.18 (t, J=7.4Hz, 2H), 2.93 (t, J=7.4Hz, 2H), 2.77 (t, J=6.4Hz, 2H), 2.18 (t, J=7.6Hz, 2H), 2.06 (dt, J=6.8, 6.8Hz, 4H), 1.60 (quint, J=7.2Hz, 2H), 1.33-1.28 (14H), 0.90 (t, J=7.0Hz, 3H) |
| 7 | Histamine-oleic acid | 7.64 (d, J=1.2Hz, 1H), 6.96 (d, J=1.2Hz, 1H), 5.39-5.28 (2H), 3.16 (t, J=7.4Hz, 2H), 2.92 (t, J=7.4Hz, 2H), 2.17 (t, J=7.8Hz, 2H), 2.08-1.96 (m, 4H), 1.60 (quint, J=7.2Hz, 1H), 1.32-1.28 (20H), 0.90 (t, J=7.0Hz, 3H) |
| 8 | Guanidine-linoleic acid | 5.39-5.28 (4H), 2.77 (t, J=6.4Hz, 2H), 2.16 (t, J=7.6Hz, 2H), 2.06 (dt, J=6.8, 6.8Hz), 1.59 (quint, J=7.2Hz, 2H), 1.33-1.29 (14H), 0.91 (t, J=7.0Hz, 3H) |
| 9 | Guanidine-oleic acid | 5.38-5.30 (2H), 2.16 (t, J=7.6Hz, 2H), 2.08-1.97 (m, 4H), 1.59 (quint, J=7.2Hz, 2H), 1.32-1.29 (20H), 0.90 (t, J=6.8Hz, 3H) |

[Table 24]

| | | 13C-NMR |
|---|---|---|
| | | Chemical shift value δ [ppm] |
| 1 | Choline-isostearic acid | 185.21, 69.06*, 69.03*, 69.00*, 57.04, 54.72*, 54.68*, 54.64*, 50.81, 34.81 (2C), 33.07 (2C), 31.04, 31.01, 30.81, 30.76, 30.48 (2C), 29.19, 23.73 (3C), 14.53 (2C) |
| 2 | Choline-linoleic acid | 182.60, 130.92, 130.90, 129.05, 129.02, 69.03*, 69.00*, 68.97*, 57.01, 54.70*, 54.66*, 54.63*, 39.34, 32.64, 30.89, 30.81, 30.57, 30.47, 30.41, 28.23, 28.17, 28.82, 26.56, 23.63, 14.54 |
| 3 | Choline-oleic acid | 182.78, 130.84, 130.77, 69.04*, 69.01*, 68.99*, 57.02, 54.71*, 54.67*, 54.63*, 39.40, 33.05, 30.90, 30.85, 30.80, 30.60, 30.57, 30.44, 30.39, 30.34, 28.20, 28.13, 27.85, 23.73, 14.54 |
| 4 | Choline-capric acid | 182.65, 69.06*, 69.03*, 69.01*, 57.03, 54.72*, 54.68*, 54.64*, 39.32, 33.05, 30.89, 30.71, 30.66, 30.45, 27.82, 23.73, 14.51 |
| 5 | Choline-benzoic acid | 175.04, 139.31, 131.28 (2C), 130.19 (2C), 128.80, 68.97*, 68.94*, 68.91*, 56.98, 54.63*, 54.59* 54.55* |
| 6 | Histamine-linoleic acid | 182.48, 136.72, 135.26, 130.91 (2C), 129.05, 129.04, 116.99, 40.52, 38.78, 32.66, 30.82, 30.79, 30.55, 30.48, 30.38, 28.23, 28.18, 27.59, 26.57, 26.34, 23.64, 14.5 |
| 7 | Histamine-oleic acid | 182.83, 136.70, 135.31, 130.84, 130.79, 117.03, 40.59, 39.07, 33.07, 30.90, 30.86, 30.84, 30.63, 30.57, 30.46, 30.38, 30.36, 28.21, 28.16, 27.72, 26.52, 23.75, 14.54 |
| 8 | Guanidine-linoleic acid | 181.73, 158.86, 129.57, 129.56, 127.70, 127.67, 37.73, 31.31, 29.48, 29.45, 29.20, 29.13, 29.04, 26.89, 26.83, 26.29, 25.22, 22.29, 13.18 |

(continued)

| 13C-NMR | | |
|---|---|---|
| | | Chemical shift value δ [ppm] |
| 9 | Guanidine-oleic acid | 183.18, 160.18, 130.84, 130.77, 39.12, 33.05, 30.88, 30.84, 30.79, 30.61, 30.54, 30.44, 30.37, 30.34, 28.20, 28.14, 27.65, 23.73, 14.54 |
| (Note) A signal that splits due to the influence of adjacent $^{14}$N was marked with *. | | |

Industrial Applicability

The transdermal absorption enhancer and the transdermal absorption enhancement aid of the present invention can be used in the use application of pharmaceutical products, cosmetics, quasi-drugs, and the like.

The present application is based on Japanese Patent Application No. 2014-221315 filed with the Japan Patent Office on October 30, 2014, the contents of which are incorporated herein by reference.

**Claims**

1. A transdermal absorption enhancement aid or a transdermal absorption enhancer comprising an equimolar salt of

   a) a basic functional group-containing compound having a molecular weight of from 50 to 120 and a melting point of from 50 to 350°C, selected from guanidine or a derivative thereof, and
   b) an acidic functional group-containing compound having a LogP of from -4 to 7.3.

2. The transdermal absorption enhancement aid or the transdermal absorption enhancer according to claim 1, wherein the LogP of the acidic functional group-containing compound b) is from 2.5 to 7.3.

3. The transdermal absorption enhancement aid or the transdermal absorption enhancer according to claim 1 or 2, wherein the acidic functional group-containing compound b) is one compound selected from the group consisting of an amino acid, a carboxylic acid, a hydroxy acid, a long-chain fatty acid having from 12 to 20 carbon atoms and optionally having a substituent, and a saccharic acid.

4. The transdermal absorption enhancement aid or the transdermal absorption enhancer according to claim 3, wherein the acidic functional group-containing compound b) is one compound selected from the group consisting of histidine, glycolic acid, lactate, malonic acid, acetic acid, maleic acid, succinic acid, glutaric acid, benzoic acid, capric acid, oleic acid, linoleic acid, and isostearic acid.

5. The transdermal absorption enhancement aid or the transdermal absorption enhancer according to claim 1, wherein the acidic functional group-containing compound b) is one compound selected from capric acid, oleic acid, linoleic acid, and isostearic acid.

6. A transdermal preparation comprising an active ingredient and a transdermal absorption enhancement aid or a transdermal absorption enhancer according to any one of claims 1 to 5.

7. The transdermal preparation according to claim 6, wherein the active ingredient has a molecular weight of 10000 or smaller and has a LogP of 3.5 or lower.

8. The transdermal preparation according to claim 6, wherein the active ingredient has a molecular weight of 10000 or smaller and has a LogP of 0.5 or lower.

9. The transdermal preparation according to any one of claims 6 to 8, wherein the active ingredient is not a salt.

10. The transdermal preparation according to any one of claims 6 to 9, wherein the active ingredient is a therapeutic drug for viral infection or an immunosuppressive agent.

Fig.1

Fig.2

Fig.3

Europäisches
Patentamt

European
Patent Office

Office européen
des brevets

## EUROPEAN SEARCH REPORT

Application Number

EP 18 17 7254

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | FR 2 752 421 A1 (FABRE PIERRE DERMO COSMETIQUE [FR]) 20 February 1998 (1998-02-20) * page 1, line 5 - line 9 * * page 2, line 14 - page 4, line 11 * * examples * | 1-10 | INV. A61K47/18 A61K47/12 A61P31/12 A61P37/06 A61K38/05 A61K31/4164 |
| X | US 5 749 947 A (GEKE JUERGEN [DE] ET AL) 12 May 1998 (1998-05-12) * column 2, line 13 - line 24 * * examples * * claims * | 1-5 | A61K31/519 A61K31/522 A61K31/7056 |
| A | JP 2008 184402 A (MEDOREKKUSU KK) 14 August 2008 (2008-08-14) * paragraph [0008] - paragraph [0013] * * examples * | 1-10 | ADD. A61K47/10 A61K47/14 A61K47/22 A61K47/26 |
| A | EP 0 189 861 A2 (SHOWA DENKO KK [JP]) 6 August 1986 (1986-08-06) * paragraph [0008] - paragraph [0013] * * examples * | 1-10 | |

TECHNICAL FIELDS SEARCHED (IPC)

A61K

The present search report has been drawn up for all claims

| Place of search | Date of completion of the search | Examiner |
|---|---|---|
| The Hague | 13 September 2018 | Marchand, Petra |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

................................................................

& : member of the same patent family, corresponding
    document

EPO FORM 1503 03.82 (P04C01)

# EP 3 395 369 A1

## ANNEX TO THE EUROPEAN SEARCH REPORT
## ON EUROPEAN PATENT APPLICATION NO.

EP 18 17 7254

This annex lists the patent family members relating to the patent documents cited in the above-mentioned European search report.
The members are as contained in the European Patent Office EDP file on
The European Patent Office is in no way liable for these particulars which are merely given for the purpose of information.

13-09-2018

| Patent document cited in search report | | Publication date | Patent family member(s) | | Publication date |
|---|---|---|---|---|---|
| FR 2752421 | A1 | 20-02-1998 | FR | 2752421 A1 | 20-02-1998 |
| | | | WO | 9806378 A1 | 19-02-1998 |
| US 5749947 | A | 12-05-1998 | AT | 180022 T | 15-05-1999 |
| | | | CA | 2202678 A1 | 25-04-1996 |
| | | | DE | 4436764 A1 | 18-04-1996 |
| | | | EP | 0786019 A1 | 30-07-1997 |
| | | | ES | 2132722 T3 | 16-08-1999 |
| | | | FR | 2725599 A1 | 19-04-1996 |
| | | | JP | H10507231 A | 14-07-1998 |
| | | | US | 5749947 A | 12-05-1998 |
| | | | WO | 9612054 A1 | 25-04-1996 |
| JP 2008184402 | A | 14-08-2008 | JP | 5117732 B2 | 16-01-2013 |
| | | | JP | 2008184402 A | 14-08-2008 |
| EP 0189861 | A2 | 06-08-1986 | NONE | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2008162899 A
- JP 4969065 B
- JP 2012233016 A
- JP 2013173805 A
- US 3551554 A
- JP 51032724 A
- JP 52083914 A
- JP 52001035 A
- JP 2193932 A
- JP 2207024 A
- JP 2014221315 A

**Non-patent literature cited in the description**

- SCEJ 2nd Three-Branch Joint Meeting. 2009
- **HATANAKA T. ; INUMA M. ; SUGIBAYASHI K. ; MORIMOTO Y.** *Chem. Pharm. Bull.,* 1990, vol. 38, 3452-3459
- **MORIMOTO Y. ; HATANAKA T. ; SUGIBAYASHI K. ; OMIYA H.** *J. Pharm. Pharmacol.,* 1992, vol. 44, 634-639